(19)

Europäisches
Patentamt
European
Patent Office
Office européen
des brevets

(11) **EP 4 451 276 A1**

(12) **EUROPEAN PATENT APPLICATION**

(43) Date of publication:
**23.10.2024 Bulletin 2024/43**

(21) Application number: **24164328.7**

(22) Date of filing: **19.03.2024**

(51) International Patent Classification (IPC):
***G16B 20/00*** (2019.01)    ***G16B 40/20*** (2019.01)
***G16H 50/20*** (2018.01)

(52) Cooperative Patent Classification (CPC):
**G16B 40/20; G16B 20/00; G16H 50/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.04.2023 IN 202321028610**

(71) Applicant: **Tata Consultancy Services Limited
Maharashtra (IN)**

(72) Inventors:
• **BOSE, TUNGADRI**
**411013 Pune - Maharashtra (IN)**
• **KAUR, HARRISHAM**
**411013 Pune - Maharashtra (IN)**

• **SINGH, RASHMI**
**411013 Pune - Maharashtra (IN)**
• **DUTTA, ANIRBAN**
**411013 Pune - Maharashtra (IN)**
• **HAQUE, MOHAMMED MONZOORUL**
**411013 Pune - Maharashtra (IN)**
• **MANDE, SHARMILA SHEKHAR**
**411013 Pune - Maharashtra (IN)**

(74) Representative: **Goddar, Heinz J.
Boehmert & Boehmert
Anwaltspartnerschaft mbB
Pettenkoferstrasse 22
80336 München (DE)**

Remarks:
The complete document including Reference
Table(s) and the Sequence Listing(s) can be
downloaded from the EPO website

(54) **METHOD AND SYSTEM FOR STRATIFICATION OF SUBJECTS AS RESPONDERS AND
NON-RESPONDERS FOR A THERAPY**

(57)    The present disclosure is related to a method
and system for stratification of subjects as one of responders or non-responders to a therapy. It is imperative
to critically evaluate the baseline/ initial microbiome
structure and composition of individuals and stratifying
them before prescribing any microbiome-based drug/ di-
etary interventions. The method identifies a panel of biological features/ indicators/ markers/ signatures that
can accurately stratify/ classify/ group individuals into responders and non-responders (for a given microbiome-based drug/ therapy) based upon the differences in
the metabolic functions of the gut microbial communities
between the baseline gut microbiome profile (i.e. before
the administration of an intervention) and after treatment
gut microbiome profile (i.e. after the administration of the
intervention). Individuals with samples showing an im-
provement in gut-health status after the administration
of the pre-biotic intervention were tagged as responders
and the rest were tagged as non-responders.

FIG. 1

**Description**

CROSS-REFERENCE TO RELATED APPLICATIONS AND PRIORITY

The present application claims priority to Indian application no. 202321028610, filed on April 19, 2023.

TECHNICAL FIELD

The disclosure herein generally relates to the field of patient stratification and, more particularly, to a method and system for stratification of subjects as one of responders or non-responders to a therapy.

BACKGROUND

Gastrointestinal tract (gut) of a human is populated by diverse communities of micro-organisms, including, archaea, viruses, protozoa, fungi, and bacteria, that live in synergy with the host. The bacteria inhabiting the gut (collectively referred as a gut microbiota) contain about 50-100 times more genes as compared to the host. The proteins/ enzymes encoded by these genes allow the gut microbiota to perform diverse metabolic/ immunological functions. Often the host is dependent on these microbiota derived proteins/ enzymes to perform one or more metabolic/ immunological functions which are essential for its health and wellbeing.

Recent advancements in sequencing technology have catalyzed microbiome research and have established a deeper understanding of host-microbiota interactions in health and disease. The administration of microbiota-targeted interventions, comprising of, probiotics/ prebiotics/ antibiotics/ synbiotics/ postbiotics, as prophylactic/ therapeutic treatments, has immense utility/ applicability in the sustenance/ maintenance of gut microbiota's function. However, despite the escalating use of the above-mentioned microbiota-targeted interventions in public healthcare and nutrition sectors, numerous experimental as well as clinical studies have established that their efficacy varies among individuals.

In clinical therapy, nutrition and healthcare sectors, the term responder corresponds to a subject/ patient/ individual/volunteer that reacts positively/ favorably to the administered treatment protocol and displays qualitative as well as quantitative changes in the health profile that suggest recovery/ improvement of the overall health status. However, responsiveness of the individual is regulated by various host-specific confounding factors, including, genetics, disease status, age, body-mass index, dietary patterns, demography and exposure to environmental stress or toxins. In addition, the gut microbiota of the individual is unique and even minute temporal imbalances/ variations in the composition of gut microbiome may alter/ modify the degree of treatment responsiveness.

The degree of responsiveness towards the administered/ investigated intervention can be predicted if the biomarkers for the therapeutic intervention's response are well elucidated. Further, the biomarkers are used for (apriori) segregation of patients in treatment category (e.g., kind of choice of breast cancer therapy based on brca1/2 mutation in the patient). Although, the biomarkers/ parameters that can be used to determine response and non-response to the treatment are complex and are often governed by various inter-connected factors. In addition, different prebiotic supplements and probiotic strains utilize distinct mechanistic properties to restore/ maintain healthy gut microbiota.

The ambiguity in the clinical response of a microbiome-targeted treatment/ drug is primarily attributed to the vast variations in the makeup of microbial communities that are as unique and individual-specific as a fingerprint. In other words, the response or non-response of microbe-based drug/ therapy (probiotics/ prebiotics/ synbiotics/ metabiotics, etc.) is differential among individuals. Numerous host-specific factors, including, age, gender, the immune system, host hormones, and co-morbidities etc., govern the response / non-response of therapy. Therefore, owing to the organism/ strain-specific/ prebiotic-specific divergent mechanism of actions, not all prebiotic/ probiotic supplements produce desired/ intended health benefits in different states of health and disease. In addition, the efficacy and response of prebiotic/ probiotic formulations is dependent on an adequate/ ideal/ optimum dosage concentration that elicits the desired health benefit in an individual or a population of individuals. As a result, selecting optimal prebiotic/ probiotic types for patients in a clinical setting is challenging.

Furthermore, random/ generalized and unsupervised administration of pre-/pro-/synbiotics to high-risk population (the population which has a higher risk of getting the disease as compared to the other population) leads to several types of adverse effects that counteract their clinical benefits. These adverse effects occur in the individuals with systemic infections and/ or with suppressed/ compromised immune system and primarily project themselves as gastrointestinal discomfort, immune tolerance against specific substances or specific bacterial strains, and increased risk of infections in individuals with prolonged hospitals or immuno-suppression.

In-vitro and in-vivo experiments have also revealed that the baseline microbiota of responders and non-responders is different, both in terms of compositional and functional profiles. Hence, for an accurate stratification of responders/ non-responders and for the mitigation of detrimental and undesired outcomes of therapy/ treatment, it is imperative to critically evaluate the baseline/ initial microbiome structure and composition of individuals and stratifying them before prescribing any microbiome-based drug/ dietary interventions.

SUMMARY

Embodiments of the present disclosure present technological improvements as solutions to one or more of the above-mentioned technical problems recognized by the inventors in conventional systems.

In an aspect, a method for apriori stratification of a subject as one of a responder or a non-responder to a therapy is provided. The method comprising: collecting a test biological sample from a subject for stratification into one of (i) a responder to a therapy and (ii) a non-responder to the therapy; extracting a microbial Deoxyribonucleic Acid (DNA), from the test biological sample, using a DNA extraction technique; performing one of (i) determining a microbial abundance of each of one or more predetermined microbes present in the test biological sample using a multiplex quantitative Polymerase Chain Reaction (qPCR) technique, from the microbial DNA, and (ii) determining the microbial abundance of each of a plurality of microbes present in the test biological sample, using from stretches of DNA sequences sequenced from the microbial DNA, to obtain a microbial taxonomic profile associated with the test biological sample; normalizing the microbial taxonomic profile associated with the test biological sample, using a data normalization technique, to obtain the normalized microbial taxonomic profile associated with the test biological sample; determining a model score using a binary classification model, based on the normalized microbial taxonomic profile associated with the test biological sample; stratifying the subj ect as one of (i) the responder to the therapy or (ii) the non-responder to the therapy, based on the model score; making a personalized recommendation to the subject for or against the therapy, based on the stratification on the responsiveness of the subject to the therapy; and determining the responsiveness of the subject to one or more therapies and guiding a best therapy among the one or more therapies based on the determined responsiveness.

In another aspect, a kit for stratification of a subject as one of (i) a responder to a therapy the (ii) a non-responder to the therapy is provided. The kit comprising: an input module for collecting a test biological sample from the subject for the stratification into one of (i) the responder to a therapy and (ii) the non-responder to the therapy; one or more hardware processors configured to analyze the test biological sample using the method; and an output module for displaying the stratification of the subject as one of (i) the responder to a therapy or (ii) the non-responder to the therapy, based on the analysis of the one or more hardware processors.

In an embodiment, the binary classification model is obtained by: collecting, a first set of training biological samples and a second set of training biological samples, from a plurality of subjects, at a first time-point and a second time-point respectively, wherein the first time-point indicates before an administration of the therapy and the second time-point indicates after the administration of the therapy; extracting, the microbial Deoxyribonucleic Acid (DNA) from each training biological sample present in the first set of training biological samples and the second set of training biological samples, using the DNA extraction technique; sequencing, the microbial DNA associated with each training biological sample present in the first set of training biological samples and the second set of training biological samples, using a sequencer, to obtain the stretches of DNA sequences associated with each training biological sample; determining, the microbial abundance of each of one or more microbes present in each training biological sample present in the first set of training biological samples and the second set of training biological samples, using the stretches of DNA sequences associated with each training biological sample, to obtain a microbial taxonomic profile associated with each training biological sample, wherein the microbial taxonomic profile comprises the microbial abundance of each of the one or more microbes corresponding to the set of microbial DNA sequences present in each training biological sample; normalizing, the microbial taxonomic profile using the data normalization technique, to obtain the normalized microbial taxonomic profile associated with each training biological sample, wherein the normalized microbial taxonomic profile comprises the normalized microbial abundance of each of the one or more microbes; obtaining a metabolic functional profile associated with each training biological sample, using the corresponding normalized microbial taxonomic profile; quantifying differences in the metabolic functional profile associated with each subject, based on the metabolic functional profile associated with each of the first set of training biological samples and the corresponding second set of training biological samples, using a gut-health score; assigning, via the one or more hardware processors, a tag to each subject of the plurality of subjects as one of: (i) the responder to the therapy if the subject is showing an improvement in a gut-health status at the second time-point as compared to the first time-point, and (ii) the non-responder to the therapy if the subject is showing a deterioration or no change in the gut-health status at the second time-point as compared to the first time-point, wherein the gut-health status is evaluated based on the corresponding gut-health score; obtaining, one or more features associated to each subject, from the corresponding microbial taxonomic profile associated with each training biological sample present in the first set of training biological samples, based on the tag assigned to each subject; and training, via the one or more hardware processors, a machine learning model, using the one or more features associated to each subject of the plurality of subjects, to obtain the binary classification model.

In an embodiment, the gut-health score is determined based on the abundance of gut microbial pathways corresponding to metabolism of one or both of beneficial metabolites or harmful metabolites at the first time-point and the abundance of the gut microbial pathways corresponding to one or more of the beneficial metabolites and the harmful metabolites at the second time-point.

In an embodiment, training the machine learning model, using the one or more features associated to each subject of the plurality of subjects, to obtain the binary classification model, comprises: (i) tagging one of a first class or a second class to each of a plurality of training biological samples obtained from the first set of training biological samples based on the assignment of the tag to each subject of the plurality of subjects as one of (a) the responder to the therapy or (b) the non-responder to the therapy; (ii) generating a training data comprising a plurality of microbial abundance profiles from the plurality of training biological samples from the first set, wherein each microbial abundance profile corresponds to each of the plurality of training biological samples and comprises of one or more features and respective abundance values, and wherein each feature in the associated microbial abundance profile corresponds to one of a plurality of microbial taxonomic groups present in the associated training biological sample; (iii) partitioning the training data into an internal training set and an internal test set, based on a predefined first parameter; (iv) randomly selecting a predefined number of subsets out of the internal training set based on a predefined second parameter, wherein each subset comprises of a randomly selected one or more features, and wherein each subset comprises a plurality of training biological samples having a proportionate part of the training biological samples belonging to the first class and the proportionate part of the training biological samples belonging to the second class; (v) noting, for each selected subset, a distribution of the abundance values of each of the features across the plurality of training biological samples in the selected subset, and the distribution of the abundance values of each of the features across the training biological samples belonging to the first class in the selected subset and the training biological samples belonging to the second class in the selected subset; (vi) calculating, from the noted distributions of each selected subset, a first quartile value $Q1$ and a third quartile value $Q3$ of the distribution of each of the features across each of the training biological samples in the selected subset; (vii) calculating, for each selected subset, a second quartile value of the distribution of each of the features across the training biological samples belonging to the first class $Q2_A$ in the selected subset and the training biological samples belonging to the second class $Q2_B$ in the selected subset; (viii) calculating $Q1$, $Q3$, $Q2_A$ and $Q2_B$ for each of a predefined number of subsets M; (ix) calculating a median value for each of the $Q1$, $Q3$, $Q2_A$ and $Q2_B$; (x) performing a Mann-Whitney test to check whether the median value ($Q2_A$) of the feature in the training biological samples belonging to the first class is significantly different (p<0.1) as compared to the median value ($Q2_B$) of the associated feature in the training biological samples belonging to the second class; (xi) shortlisting the features based on a first predefined criteria utilizing calculated median values and the Mann-Whitney test; (xii) generating a set of features using the shortlisted features using a second predefined criteria, wherein the set of features are less than or equal to a predefined second criteria value; (xiii) creating a plurality of combinations of the features present in the set of features to generate a plurality of candidate feature sets, wherein a number of the plurality of combinations of the features is equal to a minimum of two and a maximum of the predefined second criteria value; (xiv) building a plurality of candidate models ($CM_K$) corresponding to each of the plurality of candidate feature sets; (xv) calculating a model evaluation score (*MES*) corresponding to each of the plurality of candidate models; (xvi) selecting a model having a highest *MES,* out of the plurality of candidate models as a best model, based on a first threshold ($T_{max}$), wherein the selected model is tagged as a forward model; (xvii) swapping the tagging of the first class and the second class to each of the plurality of training biological samples present in the training data; (xviii) identifying and subsequently tagging the model as a reverse model by repeating the steps (ii) through (xvi) for the training data obtained after the swapping; (xix) generating a plurality of forward models and a plurality of reverse models by repeating step (ii) through (xviii) for a predefined number of times using randomly created partitions of internal training sets and corresponding internal test sets from the training data; (xx) generating an ensemble of forward models (ENS-$MD_{fwd}$) using the plurality of forward models and an ensemble of reverse models (ENS-$MD_{rev}$) using the plurality of reverse models; (xxi) identifying a best forward model and a best reverse model using the model evaluation score *(MES);* and (xxii) choosing a final single model ($FM_{single}$) from amongst the best forward models and the best reverse model, and a final ensemble classification model ($FM_{ens}$) from among the ensemble of forward models and the ensemble of reverse models, based on the classification of the individual training biological samples from the training data, as a binary classification model.

In an embodiment, training the machine learning model, using the one or more features associated to each subject of the plurality of subjects, to obtain the binary classification model, further comprises: classifying each of the set of shortlisted features using a second threshold value different from the first threshold ($T_{max}$); and cumulating the results to construct a receiver operating characteristic curve (ROC) for each of the shortlisted features, wherein an area under the curve (AUC) of the ROC is indicative of utility of the feature to distinguish between the training biological samples belonging to the first class and the second class.

In an embodiment, calculating the model evaluation score *(MES)* comprises transforming the values of the set of features as follows:

$$F_j' \; = \; 0 \ldots\ldots\ldots\ldots\ldots \text{ if } F_j < \widetilde{Q1}_j$$

$$F'_j = 1 \quad \dots\dots\dots\dots\dots \text{ if } F_j > \widetilde{Q3}_j$$

$$F'_j = 0.5 \quad \dots\dots\dots\dots\dots \text{ if } \widetilde{Q1}_j = \widetilde{Q3}_j$$

$$F'_j = \frac{F_j - \widetilde{Q1}_j}{\widetilde{Q3}_j - \widetilde{Q1}_j} \quad \dots\dots\dots\dots\dots \text{ if } \widetilde{Q1}_j < F_j < \widetilde{Q3}_j;$$

collating the features out of the set of features as a set of numerator features ($F_{numerator}$) if $\widetilde{Q2_{B_J}} > \widetilde{Q2_{A_J}}$ , else, collating the features out of the set of features as a set of denominator features ($F_{denominator}$);

constituting a ratio function for each of the candidate model as:

$$CM_K = \frac{\Sigma\, F_{numerator}}{\Sigma\, F_{denominator}} \dots \text{ when } F_{numerator} > 0 \text{ and } F_{denominator} > 0$$

or,

$$CM_K = \frac{\Sigma\, F_{numerator} + 1}{\Sigma\, F_{denominator} + 1} \dots \text{ when either } F_{numerator} \text{ or } F_{denominator} = 0$$

wherein, $\Sigma\, F_{numerator}$ represents the sum of values of all numerator features for a particular training biological sample, and,

wherein, $\Sigma\, F_{denominator}$ represents the sum of values of all denominator features for a particular training biological sample;

generating a candidate model score ($CMS_K$) for each of the training biological samples in the internal train set; removing the top 10 percentile and bottom 10 percentile scores as outliers from the set of scores $CMS_K$, and identifying maximum and minimum scores from the set $CMS_K$ as $CMS_{K_{max}}$ and $CMS_{K_{min}}$ respectively; reclassifying the training biological samples in the internal train set by considering each score in the set $CMS_K$ as threshold, such that the training biological sample is classified into the second class if $CMS_K$ is more than or equal to the threshold, or the training biological sample is classified into the first class if $CMS_K$ is less than the threshold; calculating Matthew's correlation coefficients ($MCC$) for each of the thresholds based on a comparison of the reclassified training biological sample and the original classes of the training biological samples, to evaluate how well each of the thresholds are able to distinguish between the training biological samples associated to the first class and the second class; identifying the threshold as a first threshold ($T_{max}$) which provides maximum absolute $MCC$ value; discarding the candidate models for further evaluation if the maximum absolute $MCC$ value is less than 0.4; considering the ($|MCC_{max}|$) value as the 'train-MCC' value ($MCC_{train}$) for the model $CM_K$ and the first threshold ($T_{max}$) is used to classify the training biological samples in the internal-test set; comparing the classification results on the training biological samples from the internal test set against the original classes of the training biological samples with pre-assigned labels, and the $MCC$ for the model $CM_K$ and the threshold $T_{max}$ threshold on the internal train set is calculated ($MCC_{test}$); and calculating a model evaluation score *(MES)* for candidate model $CM_K$ as: $MES = |(MCC_{train} + MCC_{test})| - |(MCC_{train} - MCC_{test})|$.

In an embodiment, training the machine learning model, using the one or more features associated to each subject of the plurality of subjects, to obtain the binary classification model, further comprises: evaluating collective classification efficiencies of the ensemble of forward models (ENS-$MD_{fwd}$) and the ensemble of reverse models (ENS-$MD_{rev}$), using an ensemble model scoring method, wherein a model scores ($MS$) corresponding to each of the ensemble is transformed into a scaled model scores ($SMS$) having values between -1 and +1, wherein,

$$SMS = (MS - T_{max})/(CMS_{K_{max}} - T_{max}), \quad \dots\dots \text{ when } MS >= T_{max},$$

and

$$SMS = (MS - T_{max})/(T_{max} - CMS_{K_{min}}), \ldots \text{ when } MS < T_{max},$$

wherein, $T_{max}$, $CMS_{K_{max}}$ and $CMS_{K_{min}}$ values corresponding to the respective model.

In an embodiment, calculating the model evaluation score (*MES*) further comprises calculating an average of all *SMS* ($SMS_{avg}$) obtained using all models in the ensemble, wherein $SMS_{avg} = SMS_{avg} * (+1)$ while using the ensemble of forward models (ENS-$MD_{fwd}$),

If $SMS_{avg} >= 0$, training biological sample is classified as the second class; and
If $SMS_{avg} < 0$, training biological sample is classified as the first class; and
$SMS_{avg} = SMS_{avg} * (-1)$ while using the ensemble of reverse model (ENS - $MD_{rev}$),
If $SMS_{avg} > 0$, training biological sample is classified as the second class; and
If $SMS_{avg} <= 0$, training biological sample is classified as the first class.

In an embodiment, the binary classification model is one of: the final single model ($FM_{single}$) or an ensemble of more than one classification models ($FM_{ens}$).

In an embodiment, the first predefined criteria is if a feature ($F_j$) is observed to have significantly (p<0.1) different median values in the first class compared to the second class in >70% of predefined number of subsets, and if $\widetilde{Q2_{A_j}} >$ $= Q2_{min}$ or $\widetilde{Q2_{B_j}} >= Q2_{min}$, $F_j$ is added to a set of shortlisted features (*SF* ).

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for stratification of a subject as one of a responder or a non-responder to a therapy, according to some embodiments of the present disclosure.
FIGS. 2A and 2B are flowcharts illustrating a method for stratification of a subject as one of a responder or a non-responder to a therapy, according to some embodiments of the present disclosure.
FIGS. 3A and 3B are flowcharts illustrating steps involved in a data preparation for building a binary classification model, according to some embodiments of the present disclosure.
FIGS. 4A, 4B and 4C are flowcharts illustrating steps involved in building a binary classification model, according to some embodiments of the present disclosure.
FIG. 5 illustrates an exemplary block diagram of a kit for stratification of a subject as one of a responder or a non-responder to a therapy, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

GLOSSARY

Microbiota: The collection of micro-organisms, such as, bacteria, archaea, protists, fungi, and virus, that inhabit a particular niche or geographical site.
Microbiome: The collection of genetic material of micro-organisms that reside in a particular geographical niche.
Probiotics: A micro-organism or a collection of micro-organisms introduced into the body for its beneficial qualities.

Prebiotics: A non-digestible food or food component that promotes the growth of beneficial micro-organisms in the gut.

Synbiotics: Synbiotics refer to foods or food components/ ingredients/ supplements that combine probiotics and prebiotics so that the beneficial effect of both is exerted in a form of synergism, hence synbiotics.

Metabiotics: Metabiotics are the structural components of probiotic microorganisms alone or in combination with their metabolites/ signaling molecules that are capable of exerting beneficial effects on host.

Biotherapeutics: Biotherapeutics are products used as drugs/ therapy candidates with active component extracted or produced by biological source.

Nutraceuticals: Nutraceuticals are food components that have a potentially positive effect on health beyond basic nutrition by promoting optimal health and by reducing risk of disease.

Responder: The term 'responder' refers to an individual/ patient/ subject who displays a favorable response to a drug/ intervention.

Non-responder: The term 'non-responder' refers to an individual/ patient/ subject who does not displays a favorable/intended response to a drug/ intervention.

Most of the commercially available microbiome-based drug / therapy are administered to general healthcare seeking population based on the assumption that similar organisms/ group of organisms are depleted, or similar metabolic functions and/or metabolites are misplaced in every individual. This assumption, along with the other complex parameters, widely misconstrues the response of microbiome-based drug/ therapy among individuals. Hence, the systematic evaluation of the structure and composition of gut microbiome prior to the prescription/ administration of any microbiome-targeted drug/ therapy can remarkably assist in accurate identification/ determination of responders and non-responders and maximize the effectiveness of the microbiome-targeted therapeutic regimens.

The measurement of response towards a pre-/ pro-/ synbiotic formulation is ascertained by different parameters and the response primarily depends on the precise objectives and endpoints of the study. Hence, an accurate categorization of health and disease status along with a correlation of various host-associated factors is crucial before enrollment of participants in a study aimed at estimating the response/ efficacy for a microbiome-based drug/ therapy. In addition, the assessment of response becomes challenging in the absence of rigorous follow-up studies. Interestingly, many studies have identified biomarkers that can reliably predict/ monitor the response for a microbiome-based drug/ therapy. However, the discovery and assessment of novel and accurate biomarkers that can be utilized to estimate response, efficacy and toxicity for an intervention require studies to be conducted for individuals belonging to multiple ethnic groups, having varied dietary preferences and gut microbiota composition, and hailing from different geographies. Besides that, majority of identified biomarkers are in early exploratory stages of development, and population data pertaining to biomarkers specific for healthy state is inadequate for translation into clinical applications. Hence, the utility of such biomarkers in a priori predicting response outcome is difficult in healthy individuals. To date, there are no universal one-size-fits-all markers/ signatures that can reliably predict or establish the response of a therapy/ medication that targets the microbiome of an individual or a group of individuals. Besides that, a low average response significantly knocks down the cost-to-benefit ratio of pre/pro/syn/meta-biotic formulations that are constructed using sophisticated industrial procedures after careful scientific deliberation and experimentation.

It is sometimes feasible to determine the response and efficacy by understanding the mechanisms through which the active ingredients/ constituents of the microbiome-based drug/ therapy interact with the body and the resident gut microbiota. One strategy to elicit an optimal response is to carefully select a prebiotic that boosts the probiotic bacterial strains that reside in the host's gut to improve the gut health and overall wellbeing of the individual.

The administration of generic prebiotic for the favorable clinical effect coupled together with the insufficient understanding of the structure and composition of host's habitual microbial communities may interfere with the metabolic functions carried out by the resident microbiota, thereby concomitantly altering the health/disease status of the host.

Estimating the response of one or more prebiotics is challenging. Several in-vitro and in-vivo studies are conducted to elucidate the mechanisms by which different kinds of prebiotics influence the activity of gut microbiota. The in-vitro studies are mostly preferred over in-vivo studies because they are faster, cheaper and more ethical as compared to the in-vivo studies. However, they fail to replicate the complex physiological and physiochemical mechanisms that occur in animal and human digestive tract. In addition, a thorough understanding of human digestion is required for replicating the complex environment of human digestive tract to achieve an ideal in-vitro experiment model with selective substrates. However, such models are not reliable as the techniques required to replicate them are difficult and can be influenced by human error. Besides that, even an ideal in-vitro model will be unable to represent the diversity of gut microbiota. Therefore, the findings of the in-vitro experiments aimed at understanding the effects and responses of different prebiotics should be validated by larger in-vivo experimentation before translating them into clinical practice.

On a different note, the estimation of Quality of Life (QoL), that qualitatively evaluates the physical, mental, and social health of the subjects/ patients can support the identification of responder and non-responder. However, QoL data, such as, anthropometrics, dietary preferences, exercise frequency, or physical activity, along with the characterization of microbiota composition, is often not exhaustively collected in clinical trials. Even in the presence of such descriptive studies, definite conclusions cannot be drawn due to poor completion rate of QoL assessment tools, including, surveys and questionnaires.

The present disclosure provides a method and system for stratification of the subject as one of a responder or a non-responder to a therapy. The method identifies a panel of biological features/ indicators/ markers/ signatures that can accurately stratify/ classify/ group individuals into responders and non-responders (for a given microbiome-based drug/ therapy) based upon the differences in the metabolic functions of the gut microbial communities between the baseline gut microbiome profile (i.e. before the administration of an intervention) and after treatment gut microbiome profile (i.e. after the administration of the intervention). Longitudinal gut microbiome samples (at two time-points) of a set of subjects from a publicly available dataset were analyzed to assess/ measure/ quantify the differences between the metabolic functions of gut microbial communities before and after a pre-biotic intervention by utilizing a gut-health score of the subject. In an example, the 'GutFeel' algorithm has been used for measuring the gut-health score. The gut-health score of the samples was computed by comparing the metabolic functional profiles of samples at the two studied time-points. The samples showing an improvement in gut-health status after the administration of the pre-biotic intervention were tagged as responders and the rest were tagged as non-responders. This information is further utilized to arrive at a set of microbial biomarkers for response to a given pre-biotic at a baseline stage (prior to pre-biotic intervention) by application of a novel supervised machine learning algorithm on the samples of individuals tagged as responders and non-responders.

The present disclosure is configured to reliably predict the response of a prebiotic intervention and accurately stratify the population by examining the composition and function of the microbes present in a stool/ fecal sample of an individual. This makes the present method relatively simple, non-invasive, cost-efficient, geography agnostic and independent of any post experimental follow-up studies. Further, the solution proposed in the present disclosure determine the responsiveness profiles of individuals towards various drugs/ interventions/ treatments, thereby facilitating the study and making of personalized/ individual-specific microbiome-based drug/ therapy. Such personalized interventions when employed in clinical settings bring a breakthrough in the future of personalized/ precision medicine and assist healthcare providers to effectively screen/ treat/ manage individuals or a group of individuals based upon their responsiveness profiles.

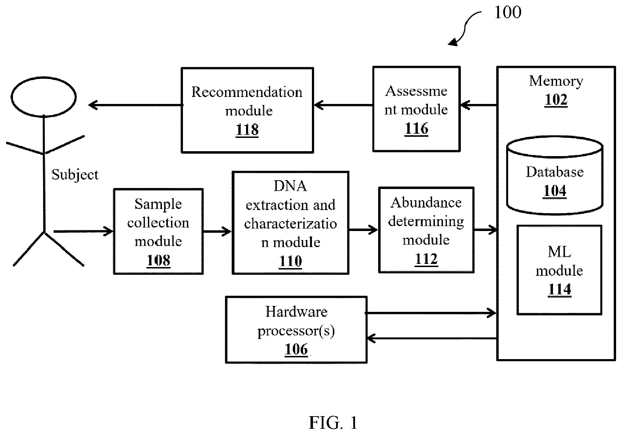

Referring now to the drawings, and more particularly to FIG. 1 through FIG. 5, where similar reference characters denote corresponding features consistently throughout the figures, there are shown preferred embodiments and these embodiments are described in the context of the following exemplary system and/or method.

FIG. 1 illustrates an exemplary block diagram of a system 100 for stratification of a subject as one of a responder or a non-responder to a therapy, according to some embodiments of the present disclosure. In an embodiment, the system 100 includes a memory 102, a database 104, one or more hardware processors 106, a sample collection module 108, a DNA extraction and characterization module 110, an abundance determining module 112, a machine learning (ML) module 114, an assessment module 116, and a recommendation module 118. In an embodiment, the database 104 and the machine learning (ML) module 114 are stored in the memory 106.

In an embodiment, the sample collection module 108 is configured to collect a biological sample of the subject whose stratification as one of the responders or the non-responders to the therapy to be assessed. The subject is a human being. The DNA extraction and characterization module 110 is configured to extract a microbial deoxyribonucleic acid (DNA) from the biological sample. The abundance determining module 112 is configured to determine a quantitative abundance of each of a plurality of predetermined microbes associated with the biological sample, to obtain a microbial taxonomic profile associated with the biological sample, using the microbial DNA.

The machine learning (ML) module 114 is configured to determine a model score based on a normalized microbial taxonomic profile which is obtained by normalizing the microbial taxonomic profile associated with the biological sample, using a data normalization technique, using a binary classification model. The assessment module 116 is configured to perform the stratification of the subject as one of (i) the responder to the therapy or (ii) the non-responder to the therapy, based on the model score. Lastly, the recommendation module 118 is configured to make a personalized recommendation for the subject based on the stratification on the responsiveness of the subject to the therapy.

In an embodiment, the one or more hardware processors 106 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 106 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 104 configured to include information regarding risk assessment of breast cancer present in the subject. The memory 102 may comprise information pertaining to input(s)/output(s) of each step performed by the one or more hardware processors 106 of the system 100 and methods of the present disclosure. In an embodiment, the database 104 may be external (not shown) to the system 100 and coupled to the system 100 via the I/O interfaces (not shown in FIG. 1).

In an embodiment, one or more data storage devices or the memory 102 operatively coupled to the one or more hardware processors 106. The system 100 with the one or more hardware processors 106 is configured to execute functions of one or more functional modules of the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

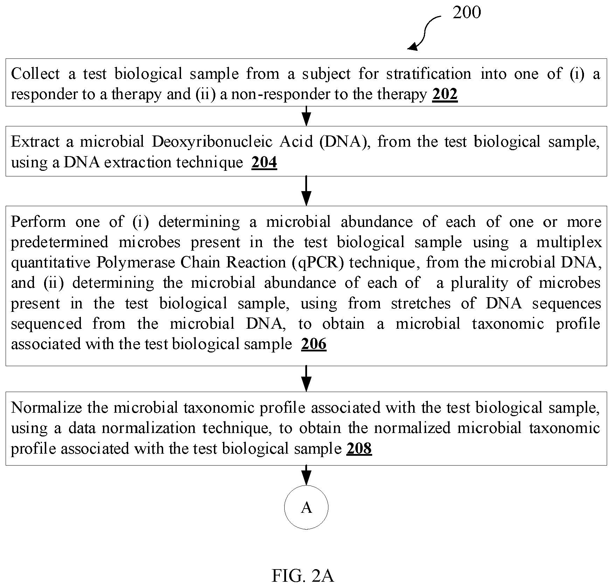

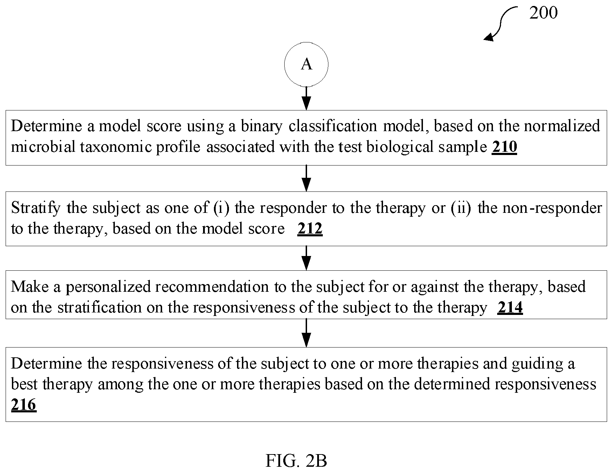

In an embodiment, the memory 102 comprises one or more data storage devices operatively coupled to the one or more hardware processors 106 and is configured to store instructions for execution of steps of the method depicted in FIGS. 2A and 2B by the one or more hardware processors 106. FIGS. 2A and 2B are flowcharts illustrating a method 200 for stratification of the subject as one of the responders or the non-responders to the therapy, according to some embodiments of the present disclosure.

The steps of the method 200 of the present disclosure will now be explained with reference to the components or blocks of the system 100 as depicted in FIG. 1 and the steps of flow diagrams as depicted in FIGS. 2A and 2B. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, a test biological sample from the subject for stratification into one of (i) the responder to a therapy and (ii) the non-responder to the therapy, is collected through the sample collection module 108. In an embodiment, the subject or the individual or a person is a human being. The biological sample is a test biological sample which is tested for determining the response to a prebiotic (Fructo-oligosaccharides (FOS) in the current implementation), prior to administration of that prebiotic.

In an embodiment, the test biological sample is a microbial sample and one of a saliva sample, stool sample, urine sample, etc. In alternate implementations, the sample collection module 108 can be modified to collect the microbiome sample from a body site/ location other than the gut e.g., saliva sample, urine sample etc. Microbial samples from healthy or diseased subjects, belonging to any geography or from any other mammalian organism are covered in the scope of this disclosure. In another implementation, any other intervention/ drug/ therapy that targets/ modifies the gut microbiome or vice-versa is under the scope of the disclosure. The biological sample is at least one of a stool sample, a gastrointestinal tract (gut) sample, a saliva sample, and a urine sample. The type of sample is collected based on the subject belonging to a specific region, geography, and ethnicity, and further based on the standard medical procedure followed in the specific region.

Further, at step 204 of the method 200, a microbial deoxyribonucleic acid (DNA) from the test biological sample collected at step 202 of the method 200, is extracted through the DNA extraction and characterization module 110.

Further, at step 206 of the method 200, determining a microbial abundance of the test biological sample is performed to obtain a microbial taxonomic profile associated with the test biological sample. One of (i) a multiplex quantitative Polymerase Chain Reaction (qPCR) technique and (ii) a DNA sequencing technique, is employed to determine the microbial abundance of the test biological sample

With the multiplex quantitative Polymerase Chain Reaction (qPCR) technique, the microbial abundance of each of one or more predetermined microbes present in the test biological sample is determined from the microbial DNA extracted at step 204 of the method 200. With the DNA sequencing technique, the microbial abundance of each of a plurality of microbes present in the test biological sample, is determined using from stretches of DNA sequences sequenced from the microbial DNA extracted at step 204 of the method 200. In an embodiment, the microbial taxonomic profile associated with the test biological sample includes a quantitative abundance value of each of the one or more predetermined microbes present in the test biological sample, if the multiplex quantitative Polymerase Chain Reaction (qPCR) technique is employed. In another embodiment, the microbial taxonomic profile associated with the test biological sample includes the quantitative abundance value of each of the plurality of microbes present in the test biological sample, if the DNA sequencing technique is employed.

More specifically in the DNA sequencing technique, stretches of DNA sequences sequenced from the microbial DNA extracted from the test biological sample, are utilized for determining the microbial abundance of each of the plurality of microbes. The DNA extraction and characterization module 110 further includes one or more DNA extraction techniques. In an embodiment, the extraction of the stretches of DNA sequences from the microbial DNA of the test biological sample is performed by amplification of 16S rRNA marker genes (either full-length or specific variable regions of the gene) using one or more of: a next-generation sequencing (NGS) platform, Oxford nanopore sequencing or any other DNA sequencing technique and platform (including a classical Sanger sequencing).

In another embodiment, the NGS platforms include any one of whole genome sequencing, CPN60 gene-based amplicon sequencing, other phylogenetically conserved genetic region-based amplicon sequencing, sequencing using approaches which involve either a fragment library or a mate-pair library or a paired-end library or a combination of the same. Further, the DNA extraction and characterization module 110 includes a set of sub-extraction modules to perform taxonomic classification of the sequenced reads at genus level using RDP, and latest version of any other taxonomic classification database such as Greengenes or Silva databases, or algorithms such as dada2 are covered in the scope of this invention.

In the DNA sequencing technique utilizes the stretches of DNA sequences sequenced from the microbial DNA extracted from the test biological sample. These techniques can include DNA sequencing, affinity chromatography or immunoprecipitation techniques based on bacterial surface moieties (proteins, glycoproteins, lipoproteins etc.), flow cytometry + qPCR/RT-PCR multiplex targeted qPCR/RT-PCR, Digital droplet PCR, nucleic acid hybridization techniques like phylo-chip etc.

In the multiplex quantitative Polymerase Chain Reaction (qPCR) technique, the quantitative abundance of each of the one or more predetermined microbes present in the test biological sample are determined. For this, a set of probes specific to each of the one or more predetermined microbes are designed in the form of layout for determining the microbial abundance. More specifically, the multiplexed quantitative Polymerase Chain Reaction (qPCR) technique define a layout and arrangement of the plurality of probes for determining the quantitative abundance associated with the test biological sample. The set of probes specific to each of the plurality of predetermined microbes associated with the test biological sample are utilized in a predefined number of a sequential multiplexed qPCR runs (defined by the multiplexed quantitative Polymerase Chain Reaction (qPCR) technique), to determine the quantitative abundance of each of the plurality of predetermined microbes associated with the test biological sample.

The microbial abundance of each of one or more predetermined microbes present in the test biological sample, is determined to obtain a microbial taxonomic profile associated with the test biological sample. The microbial taxonomic profile associated with the test biological sample, comprises microbial abundance of each of the one or more predetermined microbes corresponding to a set of microbial DNA sequences present in the test biological sample.


**Design configuration & number of multiplexed qPCR runs required for quantifying the abundance of target microbes or microbial taxonomic groups or microbial taxa/ features:**

The quantitative abundance of each of the microbial taxonomic groups or microbes, that are common to each of the multiplexed qPCR runs (the first multiplexed qPCR run, and the second multiplexed qPCR run), is determined based on a normalizing factor ($NF_{run}$) associated with each multiplexed qPCR run and the quantitative abundance of associated microbial taxonomic group in the corresponding multiplexed qPCR run.

For example, considering a maximum of five unique DNA fragments, each representing a microbial taxa or spike DNA, can be quantified in a one multiplexed qPCR run. Therefore, to analyze a disease signature (captured in an ML model) comprising of 'n' microbial taxa/ features, a minimum of $(1 + \lceil (n-4)/4 \rceil)$ multiplexed qPCR runs would be required wherein 'n' is the unique number of microbial taxonomic groups constituting the frugal set of markers, and wherein each multiplexed qPCR run is configured to determine, in the test biological sample, the relative abundance of a predetermined subset of the microbial taxonomic groups constituting the disease signature. This minimum number is based on assumptions that:

(a) the spike DNA should be analyzed at least once in one of the '$(1 + \lceil (n-4)/4 \rceil)$,' multiplexed qPCR runs; and
(b) an overlap of at least one microbial taxa/ features was done between two corresponding runs.

For example, if a disease signature comprises of 8 microbial taxa (A, B, C, D, E, F, G, and H), then at least TWO multiplexed qPCR runs would be required, where Z is the spike DNA of known concentration and taxa 'D' is analyzed in both multiplexed qPCR runs. Here, $\lceil (n-4)/4 \rceil$ indicates a ceiling value of the expression. Thus, the minimum no. of required qPCR runs would be:

1 for 1-4 signatures/ features
2 for 5-8 signatures/ features
3 for 9-12 signatures/ features
4 for 13-16 signatures/ features, and so on...

**Example A: Run 1:** Z A B C <u>D;</u> **Run2:** <u>D</u> E F G H

Similarly, for a feature size of 12 (A, B, C, D, E, F, G, H, I, J, K, and L), at least THREE multiplexed qPCR runs would be required, where Z is the spike DNA of known concentration and taxa 'D' and 'H' are analyzed in twice.

**Example B: Run 1:** Z A B C <u>D;</u> **Run 2:** <u>D</u> E F G <u>H;</u> **Run 3:** <u>H</u> I J K L

If the number of features constituting the signature is not optimal for the above condition, i.e., for e.g., the number of features is 10, then more than one microbial taxon can be analyzed twice. The same is exemplified below, wherein taxa C and D are analyzed twice (in Runs 1 and 2). Similarly, taxa F and G are also analyzed twice (in Runs 2 and 3).

**Example C: Run 1:** Z A B <u>C D;</u> **Run 2:** <u>C D</u> E <u>F G;</u> **Run3:** <u>F G</u> H I J

In alternate implementations, the spike DNA (Z) can be analyzed in each of the runs. In that scenario, the first multiplexed qPCR will be able to accommodate up to FOUR features. Each additional multiplexed qPCR run will accommodate up to THREE new/ additional features as shown by underlining in the example below. Thus, two multiplexed qPCR runs would be required for a feature set of up to seven; three qPCR runs for a feature set of up to ten and so on.

**Run 1: Z** A B C <u>D</u> ; **Run 2: Z D** <u>E F G</u> ; **Run 3: Z G** <u>H I J</u>

Furthermore, if the number of features is not optimal for the above condition, then two or more taxa/ features can be analyzed multiple times as shown in example C.

**Methodology to interpret/ quantify the abundance of a microbial taxon or microbes or microbial taxonomic groups from data obtained from above qPCR configurations:**

Given that the concentration of the spike DNA (Z) is previously known - say $X_1$. If the measured concentration of Z in the multiplexed qPCR is $X_2$, then all the measured concentration n in a single multiplexed qPCR run can be normalized multiplying by a normalizing factor ($NF_{run}$) of $X_1/X_2$.

In cases where the spike DNA is only analyzed in only one of the multiplexed qPCR runs (as shown in examples A, B and C), then the normalized values of the taxa/ feature in the first run which is/ are re-analyzed in the Run 2, can be used for adjusting the concentrations inferred from the Run 2 of the multiplexed qPCR. Following Example-A (described previously),

Actual conc of Z: $X_1$
Measured conc of Z: $X_2$
Normalizing factor $NF_{run1}$: $X_1/X_2$
Inferred conc. of A (from Run 1): $A'_{run1} \times NF_{run1}$
Inferred conc. of B (from Run 1): $B'_{run1} \times NF_{run1}$
Inferred conc. of C (from Run 1): $C'_{run1} \times NF_{run1}$
Inferred conc. of D (from Run 1): $D'_{run1} \times NF_{run1}$

Where $A'_{run1}$, $B'_{run1}$, $C'_{run1}$, and $D'_{run1}$ are the measured/ analyzed concentrations of taxa/ feature A, B, C and D respectively.
Normalizing factor $NF_{run2}$: Inferred conc. of D from Run 1/ Measured concentrations of feature D in Run 2

Inferred conc. of E: $E'_{run1} \times NF_{run2}$
Inferred conc. of F: $F'_{run1} \times NF_{run2}$
Inferred conc. of G: $G'_{run1} \times NF_{run2}$
Inferred conc. of H: $H'_{run1} \times NF_{run2}$

The same protocol may be repeated for normalizing/ adjusting the concentrations measured from all subsequent runs (as in example B). In case wherein more than once feature is analyzed in subsequent runs (as in example C), a median Normalizing factor *(NF)* - derived from the *NFs* for each of the replication features may be used for computing the inferred concentrations from that run.

In alternate implementations, wherein the spike DNA (Z) is analyzed in each of the runs (as in example D), Normalizing factor *(NF)* corresponding to each of the runs may be computed and used for inferring the concentrations of the constituent features. In cases, where the measured spike DNA (Z) concentration varies by more than 25% from the actual concentration, it is suggested that the observations from the said multiplexed qPCR run be discarded, and a fresh multiplexed qPCR run for the sub-set of features be performed.

In an alternate implementation using multiplexed qPCR runs, the marker feature (marker microbe or taxa) having the lowest variance in relative abundance in training data across both the classes, is selected as the anchor marker *(AM)*, and the relative abundance of each of the markers is computed by multiplying the ratio of their estimated/inferred DNA concentrations and the estimated/inferred DNA concentration of *AM* with the median abundance of *AM* across all training data. For example, if the marker features are A, B, C and D. wherein A is the anchor marker *(AM)* having a median abundance of $ABN_{AM}$, then the abundances of the marker features B, C and D will be computed as;

$$ABN_B = (Inferred\ conc.\ of\ B\ /\ Inferred\ conc.\ of\ A) \times ABN_{AM}$$

$$ABN_C = (Inferred\ conc.\ of\ C\ /\ Inferred\ conc.\ of\ A) \times ABN_{AM}$$

$$ABN_D = (Inferred\ conc.\ of\ D\ /\ Inferred\ conc.\ of\ A) \times ABN_{AM}$$

In an embodiment, a multiplex quantitative Polymerase Chain Reaction (qPCR) runs is employed when there are more than four microbes whose microbial abundance to be determined in the test biological sample.

Further, at step 208 of the method 200, the microbial taxonomic profile associated with the test biological sample, is normalized using a data normalization technique, to obtain a normalized microbial taxonomic profile associated with the test biological sample. In an embodiment, the normalized microbial taxonomic profile associated with the test biological sample comprises a normalized microbial abundance value of each of the one or more predetermined microbes, if the multiplex quantitative Polymerase Chain Reaction (qPCR) technique is employed at step 206 of the method 200. In another embodiment, the normalized microbial taxonomic profile associated with the test biological sample comprises the normalized microbial abundance value of each of the plurality of microbes, if the DNA sequencing technique is employed at step 206 of the method 200. In an embodiment, the data normalization technique is used to normalize the microbial abundance value of each of the microbes in a predefined normal scale.

At step 210 of the method 200, a model score is computed, through the ML module 114, based on the normalized microbial taxonomic profile associated with the test biological sample obtained at step 208 of the method 200. The ML module 114 includes a binary classification model is employed for determining the model score. More specifically, the predefined set of features that predict binary response of the therapy is filtered using the binary classification model from the normalized abundance profile of the test biological sample.

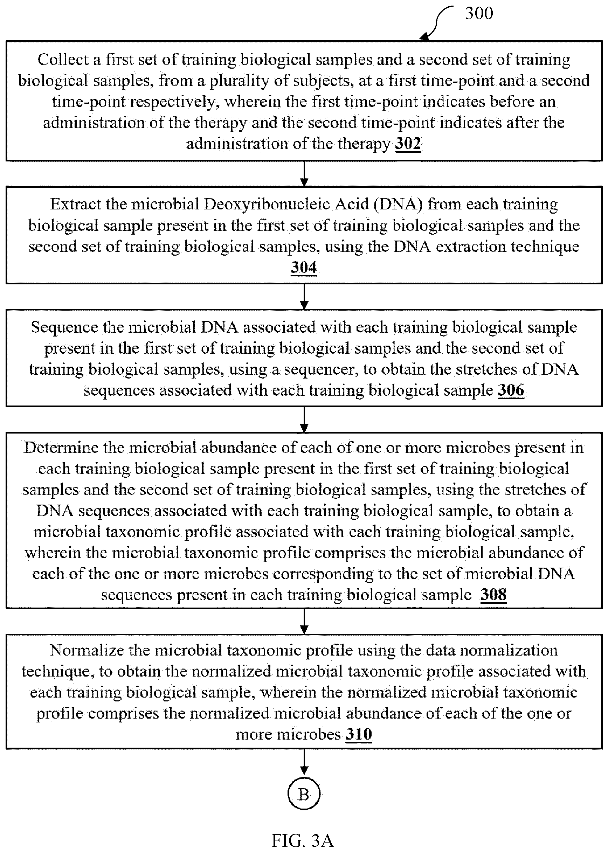

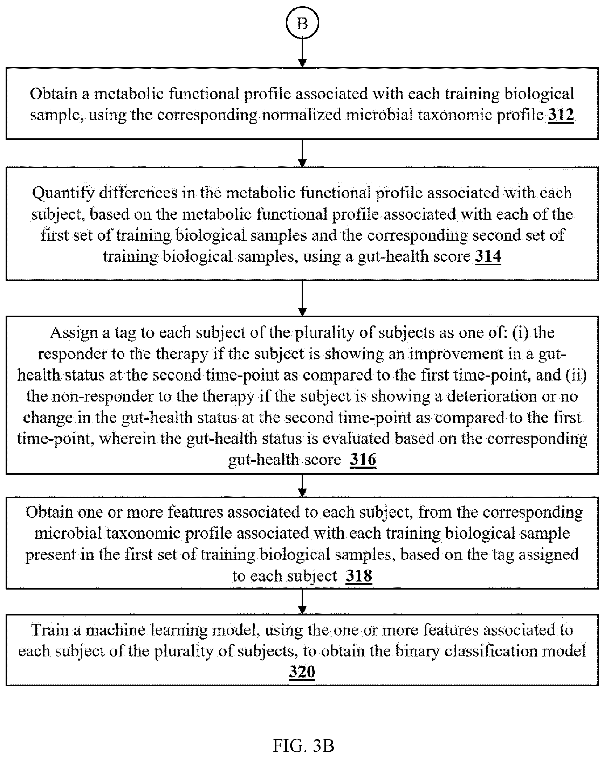

FIGS. 3A and 3B are flowcharts illustrating steps involved in a data preparation 300 for building a binary classification model, according to some embodiments of the present disclosure. The steps are explained in detail from step 302 to step 320. Initially at step 302, a first set of training biological samples at a first time-point (Time-point 1: TP1) and a second set of training biological samples a second time-point (Time-point 2: TP2), are collected from a plurality of subjects. The first time-point (TP1) indicates before the administration of the therapy and the second time-point (TP2), indicates after the administration of the therapy. The type of the training biological samples in each first set and the second set are same as mentioned at step 202 of the method 200.

The samples are collected from the multiple subjects belonging to a specific region, geography, ethnicity, or a custom group of individuals at two time-points, i.e., before the administration of a therapy/ drug (TP1) and after the administration of the therapy/drug (TP2) to determine their response to a prebiotic. In another embodiment, the sample collection module 102 is modified to collect the biological samples from a body site/ location other than the gut e.g., saliva sample, urine sample etc. Microbial samples from healthy or diseased subjects, belonging to any geography or from any other mammalian organism are covered in the scope of the present disclosure. In another implementation, any other intervention/ drug/ therapy that targets/ modifies the gut microbiome or vice-versa is under the scope of the present disclosure.

At step 304, the microbial Deoxyribonucleic Acid (DNA) from each training biological sample present in the first set of training biological samples and the second set of training biological samples, is extracted using the DNA extraction technique as described at step 204 of the method 200.

At step 306, the microbial DNA associated with each training biological sample present in the first set of training biological samples and the second set of training biological samples, is sequenced, using a sequencer, to obtain the stretches of DNA sequences associated with each training biological sample. The DNA sequencing technique described at step 206 of the method 200 is used to obtain the stretches of DNA sequences associated with each training biological sample.

At step 308, the microbial abundance of each of one or more microbes present in each training biological sample present in the first set of training biological samples and the second set of training biological samples, are determined using the respective stretches of DNA sequences associated with each training biological sample, to obtain a microbial taxonomic profile associated with each training biological sample. The microbial taxonomic profile comprises the microbial abundance value of each of the plurality of microbes corresponding to the set of microbial DNA sequences present in each training biological sample. The DNA sequencing technique described at step 206 of the method 200 are used to obtain the microbial taxonomic profile associated with each training biological sample.

At step 310, the microbial taxonomic profile associated with each training biological sample, is normalized using the data normalization technique, to obtain the normalized microbial taxonomic profile associated with each training biological sample. The normalized microbial taxonomic profile comprises the normalized microbial abundance value of each of the plurality of microbes. The normalization is done by creating abundance/ feature table and generation of the normalized abundance/ feature table having percent normalized abundance values of taxa in each sample, using the predefined normal scale as described at step 208 of the method 200. Alternatively, any other pre-processing methods or data normalization techniques known in the state of art can be used for normalization and feature selection from the main feature table.

At step 312, a metabolic functional profile associated with each training biological sample is obtained, using the corresponding normalized microbial taxonomic profile.

At step 314, the differences in the metabolic functional profile associated with each training biological sample is quantified at two time-points (TP1 and TP2), using a gut-health score. The gut-health score is determined based on the abundance of gut microbial pathways corresponding to metabolism of one or both of beneficial metabolites or harmful metabolites at the first time-point TP1 (before administering the therapy) and the abundance of the gut microbial pathways corresponding to one or both of the beneficial metabolites and the harmful metabolites at the second time-point TP2 (after administering the therapy).

In an embodiment, the training biological samples corresponding to a baseline time-point (before administration of prebiotic) and after 90th day of prebiotic administration are used from a prior literature to ascertain the improvement/ deterioration/ stability of gut-health of the individuals by comparing the two time-points TP1 and TP2 using a GutFeel algorithm (Anand *et al.,* 2021). The GutFeel algorithm determines the gut-health status of any gut microbiome sample by evaluating the differences in the metabolic functional profiles of gut microbial communities at two time-points TP1 and TP2. The metabolic functional profiles of the gut microbiome samples are constructed using the abundance of bacteria that produce/ bio-synthesize certain beneficial or harmful metabolites. The differences in the metabolic functional profiles of the gut microbiome samples at two time-points TP1 and TP2 are quantified using a gut-health score/ index which is the ratio of abundance of gut microbial pathways corresponding to either beneficial metabolites or harmful metabolites or both at the time-point before the administration of any therapy and the abundance of gut microbial pathways corresponding to either beneficial metabolites or harmful metabolites or both at the time-point after the administration of any therapy. Based upon a threshold value of gut-health score, the time-point showing an improvement/ deterioration/ stability of gut-health of an individual/ subject is ascertained. The GutFeel algorithm provides an accurate and reliable method to predict the gut-health status of an individual as it utilizes microbial metabolic function-based metric which are agnostic to the differences in diet, geography, ethnicity, health status, etc., and are robust indicators which function well despite the differences in the type and composition of gut microbiome.

At step 316, a tag is assigned to each subject of the plurality of subjects considered at step 302, as one of: (i) the responder to the therapy if the subject is showing an improvement in a gut-health status at the second time-point TP2 as compared to the first time-point TP1, and (ii) the non-responder to the therapy if the subject is showing a deterioration or no change in the gut-health status at the second time-point TP2 as compared to the first time-point TP1. The gut-health status is evaluated based on the corresponding gut-health score.

In the context of the present disclosure, the gut microbial samples corresponding to first baseline time-point (referred as Time-point 1 or TP1 or the first time-point before the administration of a prebiotic) and the second baseline time-point after 90th day of prebiotic administration (referred as Time-point 2 or TP2 or the second time-point) from 52 subjects provided in the source study, are analyzed for their gut-health score using the GutFeel algorithm. Based upon the calculated gut-health score, the subjects showing an improvement in the gut-health status at TP2 were tagged as responder and those showing a deterioration or no change in the gut-health status at TP2 were tagged as non-responder.

It should be appreciated that any other method to estimate/ predict the gut-health status of an individual is within the scope of the disclosure. The presence of certain beneficial/ harmful metabolites in fecal matter is used to profile metabolic repertoire of the gut microbiota and thus estimate gut-health. The metabolic functional profile of gut microbiome can be evaluated by methods like homology search of genes/ enzymes/ protein domains of microbial pathways responsible for biosynthesis of harmful or beneficial metabolites, active-site prediction, structure-based pathway prediction, etc.

At step 318, one or more features associated to each subject, are obtained from the corresponding microbial taxonomic profile associated with each training biological sample present in the first set of training biological samples, based on the tag assigned to each subject at step 316. The tagging is utilized to label a baseline microbial taxonomic profile at the first time-point TP1 for the plurality of subjects. The features are then filtered from the baseline microbial taxonomic profile.

At step 320, a machine learning model, is trained using the one or more features associated to each subject of the plurality of subjects, to obtain the binary classification model. The binary classification model is generated using the normalized abundance of taxa/ features at only 'base line time-point' TP1 (before administration prebiotic). The taxonomic features used in the Gutfeel algorithm are filtered from this feature table (to remove any bias) and used to build the binary classification model, adopting a supervised machine learning algorithm. This binary classification model can categorize the biological sample into one of the responders and the non-responders.

In another embodiment of the present disclosure, usage of any other machine learning/ deep learning algorithms such as Random Forests, Neural Networks etc and usage of any alternate feature engineering and feature selection algorithms and/ or alternate data transformation techniques known in the state of art are covered under the scope of this disclosure. The step 302 to 320 is the one-time process, and the obtained binary classification model can categorize the biological sample into one of the responder and the non-responder.

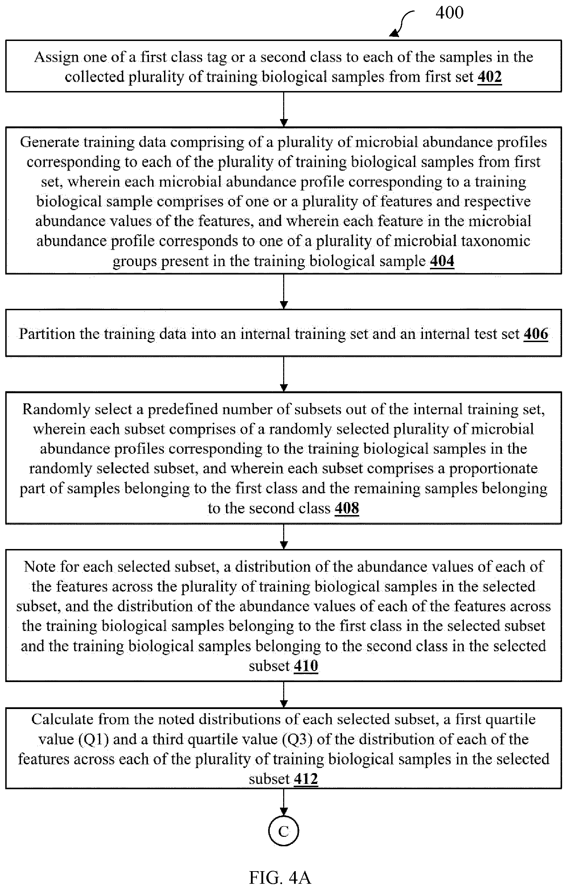

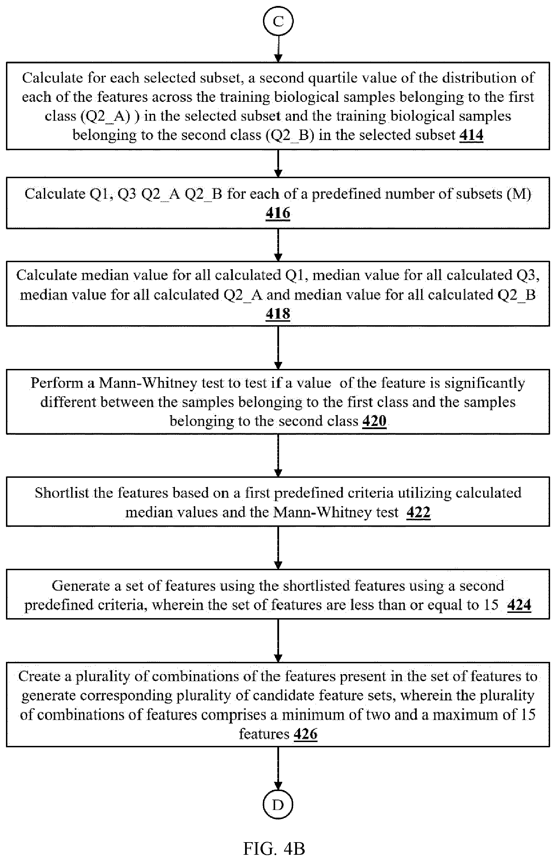

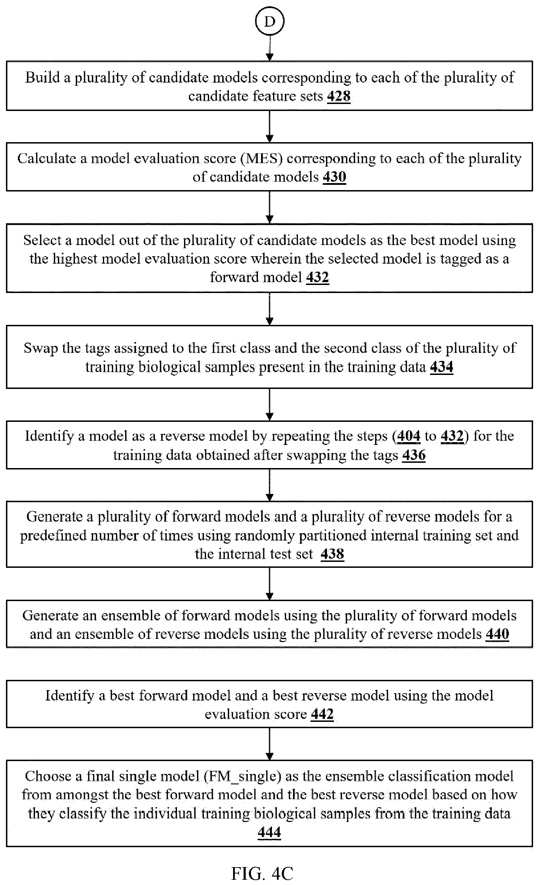

The binary classification model is an ensemble machine learning (ML) model which is built using the first set of training biological samples at the first time-point TP1. FIGS. 4A, 4B and 4C are flowcharts illustrating steps 400 involved in building the binary classification model, according to some embodiments of the present disclosure.

The technique for building the ensemble ML model accepts data in form of a feature table for multiple observations (the set of taxonomic features and their abundance values corresponding to the plurality of training biological samples in the first set of TP1) wherein each observation/ training biological sample is defined by '$N$' features ($F$) which are either or both of continuous and counted variables with ($N \geq 1$). In case of training data ($TR$), each of the training biological samples/observations further have a preassigned class/category which is binary in nature, i.e., a first class (A) (e.g., affiliating to biological samples associated with the subjects who are the responder to the therapy) and a second class (B) (e.g., affiliating to biological samples associated with the subjects who are the non-responder to the therapy). In case of test data ($TS$) or data received during actual deployment of the method, the model(s) built based on training data predicts the class/category of the test biological samples/observations. During training process, the following steps are followed:

Initially at step 402, a first class tag or a second class tag is assigned to each of the training biological samples obtained from the first set of training biological samples at first time-point (TP1) based on the assignment of the tag at step 316, to each subject of the plurality of subjects as one of (i) the responder to the therapy or (ii) the non-responder to the therapy.

At step 404, the training data comprises of a plurality of microbial abundance profiles corresponding to each of the collected plurality of training biological samples from the first set is generated. Each microbial abundance profile is generated from corresponding training biological sample and comprises of one or a plurality of feature (s) and respective abundance value (s) of the feature (s). Each feature in the microbial abundance profile corresponds to one of a plurality of microbial taxonomic groups present in the plurality of training biological samples.

In the next step 406, the training data (TR) is randomly partitioned into two sets - namely, an internal-train (*ITR*) and an internal-test (*ITS*), based on a parameter '$L_1$', wherein $L_1$% training biological samples from the total training data constitute the *ITR* set and (100 - $L_1$) % of the training biological samples constitute the *ITS* set. Furthermore, the random partitioning into *ITR* and *ITS* sets is performed using a stratified sampling approach with the intent of preserving the relative proportion of training biological samples belonging to the first class (A) or the second class (B) in the total training data in these newly drawn subsets.

In the next step 408, a predefined number of subsets are randomly selected out of the internal training set based on a second parameter ($L_2$). Each of the subset comprises a randomly selected plurality of microbial abundance profiles corresponding to the plurality of training biological samples in the randomly selected subset, and wherein each of the subset comprises a proportionate part of training biological samples belonging to the first class (A) and the remaining training biological samples belonging to the second class (B). Thus, from *ITR*, '$M$' randomly drawn subsets $ITRS_i$ (e.g., $ITRS_1$, $ITRS_2$, $ITRS_3$ .... $ITRS_M$), each containing $S$ training biological samples are further generated, wherein $S = L_2$% of the training biological samples present in *ITR*. For example, the values of $L_2$ and $M$ are 80 % and 100 respectively for present disclosure. Other values are within the scope of this invention.

In the next step 410, for each selected subset, a distribution of the abundance values of each of the features across the plurality of training biological samples in the selected subset, and the distribution of the abundance values of each of the features across the training biological samples belonging to the first class (A) in the selected subset and the training biological samples belonging to the second class (B) in the selected subset are noted. Thus, from each subset $ITRS_i$ (where $i$ = 1, 2, 3, ..., $M$), wherein there are total S training biological samples, each of which are described by N features ($F_j$) (where $j$ = 1, 2, 3, ..., $N$), the distributions of each of the features ($ITRS_iDF_j$) across S training biological samples are noted. Similarly, from each subset $ITRS_i$, wherein there are $S_A$ training biological samples belonging to the first class (A) and $S_B$ training biological samples belonging to the second class (B), each of the training biological samples being described by $N$ features ($F_j$; $j$ = 1, 2, 3, ..., $N$), the distributions of each of the features ($ITRS_iD_AF_j$) across $S_A$ training biological samples, and the distributions of each of the features ($ITRS_iD_BF_j$) across $S_B$ training biological samples are noted.

In the next step 412, from the noted distributions of each selected subset, a first quartile value ($Q1$) and a third quartile value ($Q3$) of the distribution of each of the features is calculated across each of the plurality of training biological samples in the selected subset. In an example, the respective first quartile value ($Q1$) and the third quartile value ($Q3$) of $ITRS_iDF_j$ may also be referred as $Q1ITRS_iDF_j$ and $Q3ITRS_iDF_j$.

Furthermore, in the next step 414, for each selected subset, a second quartile value of the distribution of each of the features across the training biological samples belonging to the first class ($Q2_A$) in the selected subset and the training biological samples belonging to the second class ($Q2_B$) in the selected subset is calculated. Thus, in an example, the median value (in other words, the second quartile value) of ($ITRS_iD_AF_j$) is referred as $Q2ITRS_iD_AF_j$, and the median value of ($ITRS_iD_BF_j$) is referred as $Q2ITRS_iD_BF_j$.

In the next step 416, for the M subsets of $ITRS_j$, a total of M values for each of $Q1ITRS_iDF_j$, $Q3ITRS_iDF_j$, $Q2ITRS_iD_AF_j$, and $Q2ITRS_iD_BF_j$, are calculated. Further at step 418, median value ($\widetilde{Q1}_J$) is calculated for all calculated $Q1$, median value ($\widetilde{Q3}_J$) is calculated for all calculated $Q3$, median value ($\widetilde{Q2_A}_J$) is calculated for all calculated $Q2_A$ and median value ($\widetilde{Q2_B}_J$) is calculated for all calculated $Q2_B$. Thus,

$$\widetilde{Q1}_J = \text{median of } \{Q1ITRS_1DF_j, Q1ITRS_2DF_j, Q1ITRS_3DF_j, ... Q1ITRS_MDF_j\}$$

$$\widetilde{Q3}_J = \text{median of } \{Q3ITRS_1DF_j, Q3ITRS_2DF_j, Q3ITRS_3DF_j, ... Q3ITRS_MDF_j\}$$

$$\widetilde{Q2_A}_J = \text{median } \{Q2ITRS_1D_AF_j, Q2ITRS_2D_AF_j, Q2ITRS_3D_AF_j, .... Q2ITRS_MD_AF_j\}$$

$$\widetilde{Q2_B}_J = \text{median } \{Q2ITRS_1D_BF_j, Q2ITRS_2D_BF_j, Q2ITRS_3D_BF_j, .... Q2ITRS_MD_BF_j\}$$

(where $i$ = 1,2,3, ..., M; and $j$ = 1,2,3, ...,$N$)

In the next step 420, a Mann-Whitney test is performed to test if the median value of the feature ($F_j$) is significantly ($p<0.1$) different between the training biological samples belonging to the first class ($S_A$) and the training biological samples belonging to the second class ($S_B$) in each of the $M$ randomly drawn subsets $ITRS_j$. Other statistical tests based on the nature of distribution (e.g., t-test for normal distribution), nature of sampling (e.g., Wilcoxon signed rank test for paired case and control samples) or other methods of statistical comparison relevant for microbiome datasets (e.g., ALDEx2) can also be adopted.

In the next step 422, the features are shortlisted based on a first predefined criteria utilizing calculated median values and the Mann-Whitney test. The first predefined criteria comprises if a feature $F_j$ is observed to have significantly ($p<0.1$) different values in $S_A$ compared to $S_B$ in more than 70% of M subsets, and if $\widetilde{Q2_A}_J >= Q2_{min}$ OR $\widetilde{Q2_B}_J >= Q2_{min}$ (a predefined feature 'abundance' threshold and $Q2_{min}$ threshold as described in the case study). $F_j$ is added to a set of shortlisted features (SF).

In the next step 424, a set of features is generated using the shortlisted features (SF) using a second predefined criteria, wherein the set of features are less than or equal to 15. If the number of shortlisted features (SF) obtained in previous step satisfies the criteria $1 \le SF \le 15$, then the training process proceeds to model building with all the features in SF. If no shortlisted features (SF) are obtained in previous (i.e., $SF < 1$) then following step is performed with all the

features $F_j$ for evaluating the ability of the features, when considered independently, to distinguish between training biological samples belonging to the first class (A) and the second class (B). Similarly, if the number of shortlisted features ($SF$) obtained in previous step exceeds fifteen ($SF > 15$) then following step is performed with all the shortlisted features ($SF$) for evaluating the ability of the features, when considered independently, to distinguish between the training biological samples belonging to the first class (A) and the second class (B).

Steps for shortlisting the features in case of $SF < 1$ or $SF > 15$: For each of the features (obtained previously) taken individually, different threshold values are used to classify the samples belonging to the set *ITR,* and the results are cumulated to construct a receiver operating characteristic curve (ROC curve) for each of the features. The area under the curve (AUC) of the ROC curve of any feature (AUC$^F$) is indicative of the utility of the feature to distinguish between the training biological samples belonging to the first class (A) and the second class (B), and the same is computed for every feature. The shortlisted features ($SF$) set is modified to include only the top fifteen features from a list of features arranged in a descending order of the AUC$^F$ values.

In the next step 426, a plurality of combinations of the features present in the set of features is created to generate corresponding plurality of candidate feature sets (CF), wherein the plurality of combinations of features comprises a minimum of one and a maximum of 15 features. In an embodiment, the maximum possible candidate feature sets that can be created in this process is K = $2^{15}$ - 1 = 32767 (i.e., maximum value of K = 32767).

In the next step 428, a plurality of candidate models is built corresponding to each of the plurality of candidate feature sets. At step 430, a model evaluation score *(MES)* is calculated corresponding to each of the plurality of candidate models. For each candidate feature set $CF_K$, a corresponding candidate model $CM_K$ is built and evaluated as mentioned in the steps mentioned below.

Steps for evaluating the candidate model:

- Step 1: The values of the features $F_j$ constituting a candidate feature set defining the training biological samples in *ITR* are transformed to $F'_j$ such that $-\widetilde{Q1}_J$, $\widetilde{Q3}_J$, $\widetilde{Q2_{A_J}}$ and $\widetilde{Q2_{B_J}}$

$$F'_j = 0 \dots\dots\dots\dots\dots \text{ if } F_j < \widetilde{Q1}_J$$

$$F'_j = 1 \dots\dots\dots\dots\dots \text{ if } F_j > \widetilde{Q3}_J$$

$$F'_j = 0.5 \dots\dots\dots\dots\dots \text{ if } \widetilde{Q1}_J = \widetilde{Q3}_J$$

$$F'_j = \frac{F_j - \widetilde{Q1}_J}{\widetilde{Q3}_J - \widetilde{Q1}_J} \dots\dots\dots\dots\dots \text{ if } \widetilde{Q1}_J < F_j < \widetilde{Q3}_J$$

- Step 2: If for a feature $F_j$, it is observed that $\widetilde{Q2_{B_J}} > \widetilde{Q2_{A_J}}$, then the feature $F_j$ is tagged as a 'numerator' feature and added to a set of numerator features $F_{numerator}$. Else, feature $F_j$ is tagged as a 'denominator' feature and added to a set of denominator features $F_{denominator}$
- Step 3: Each candidate model ($CM_K$) is constituted as a simple ratio function given below -

$$CM_K = \frac{\sum F_{numerator}}{\sum F_{denominator}} \dots \text{ when } F_{numerator} > 0 \text{ and } F_{denominator} > 0$$

or,

$$CM_K = \frac{\sum F_{numerator} + 1}{\sum F_{denominator} + 1} \dots \text{ when either } F_{numerator} \text{ or } F_{denominator} = 0$$

wherein, $\Sigma F_{numerator}$ represents the sum of values of all numerator features for a particular sample, and,

wherein, $\Sigma\, F_{denominator}$ represents the sum of values of all denominator features for a particular sample.

$$F_j'$$

For each of the features, a transformed value $F_j'$ as obtained above is used in the candidate model equation.

- Step 4: A candidate model c is used to generate candidate model scores ($CMS_K$) for each of the samples in the set *ITR*. From the set of scores $CMS_K$, the top 10 percentile and bottom 10 percentile scores are removed as outliers and thereafter the maximum and minimum scores from the set $CMS_K$ are noted as $CMS_{K_{max}}$ and $CMS_{K_{min}}$ respectively.
- Step 5: Considering each of the scores in the set $CMS_K$ as a threshold ($T$), the model $CM_K$ is used to (re)classify the samples in the training set (*ITR.*) such that -

  ○ the training biological sample is classified into the first class (A) if $CMS >= T$
  ○ or the training biological sample is classified into the second class (B) *if CMS < T*

  and based on a comparison of these classifications and the true/original classes of the training biological samples, Matthew's correlation coefficients (*MCC*) for each of the thresholds are calculated, to evaluate how well each of the thresholds can distinguish between training biological samples between the first class (A) and the second class (B).
- Step 6: The threshold ($T_{max}$) which provides the maximum absolute *MCC* value ($|MCC_{max}|$) is noted. If $|MCC_{max}| < 0.4$ for a candidate model $CM_K$, then the candidate model is discarded from further evaluation. Else, the $|MCC_{max}|$ value is considered as the 'train- *MCC*' value ($|MCC_{train}|$) for the model *ITS* and the model and its corresponding $T_{max}$ threshold is used to classify the training biological samples in the internal-test set (*ITS*). In another implementation of the process, the $MCC_{max}$ threshold may not be applied for retaining the candidate model for subsequent evaluation. Before classifying the each of the training biological samples in the *ITS* set, the values of features characterizing the training biological samples of the *ITS* set are transformed using the method mentioned in step

  $$\widetilde{Q1}_J, \quad \widetilde{Q3}_J, \widetilde{Q2_{A_J}} \qquad \widetilde{Q2_{B_J}}$$

  418 while using the earlier obtained values of $\widetilde{Q1}_J$, $\widetilde{Q3}_J$, $\widetilde{Q2_{A_J}}$ and $\widetilde{Q2_{B_J}}$ from the *ITR* set.
- Step 7: The classification results on the training biological samples from the *ITS* set are compared against the true/original classes of the training biological samples (with pre-assigned labels), and the *MCC* for the model $CM_K$ and its corresponding $T_{max}$ threshold on the *ITS* samples is calculated ($MCC_{test}$).
- Step 8: A model evaluation score *(MES)* for candidate model $CM_K$ is calculated as $MES = |(MCC_{train} + MCC_{test})| - |(MCC_{train} - MCC_{test})|$

In the next step 432, the model $CM_K$ is tagged as a "strong model" if all the features in the corresponding candidate feature set satisfies the Mann-Whitney test based shortlisting criteria described above. Otherwise, if any of the features in the corresponding feature set fails to satisfy the Mann-Whitney test, the model $CM_K$ is tagged as a "weak model".

Further, the above process is repeated for candidate models and respective *MES* scores are used to rank all the models. The best model is subsequently chosen based on the *MES* score. In case there are more than one model with the best *MES* score, the best model is chosen based on the following criteria (in order of preference):

  (a) the model with fewer number of features (i.e., based on a smaller candidate feature set) is chosen.
  (b) the model with lower $T_{max}$ (threshold value) is chosen.

Further, the best model obtained through above steps is tagged as a forward model ($MD_{fwd}$). The model $MD_{fwd}$

$$\widetilde{Q1}_J, \quad \widetilde{Q3}_J, \quad \widetilde{Q2_{A_J}}$$

additionally constitutes its corresponding $T_{max}$ threshold, the $CMS_{K_{max}}$ and $CMS_{K_{min}}$ values, and the

$$\widetilde{Q2_{B_J}}$$

and $\widetilde{Q2_{B_J}}$ values corresponding to the *ITR* set.

In the next step 434, the tagging of the first class (A) and the second class (B) to each of the plurality of samples present in the training data is swapped (A <-> B). At step 436, the steps 404 to 432 are repeated to determine the best model are repeated after swapping the class tags (A <-> B) for the entire training set (*TR*) to obtain a best model tagged as the reverse model ($MD_{rev}$). The model ($MD_{rev}$) additionally constitutes its corresponding $T_{max}$ threshold, the $CMS_{K_{max}}$

$$\widetilde{Q1}_J, \ \widetilde{Q3}_J, \ \widetilde{Q2_{A_J}} \qquad \widetilde{Q2_{B_J}}$$

and $CMS_{K_{min}}$ values, and the and values corresponding to the *ITR* set.

At step 438, a plurality of forward models and a plurality of reverse models are generated by repeating step (404) through (436) for a predefined number of times using randomly partitioned internal training set and the internal test set. The steps (404) through (436) are iterated 'R' times using multiple randomly partitioned *ITR* and *ITS* sets generated initially. After each iteration, (i) the features constituting the models $MD_{fwd}$ and the models $MD_{rev}$ obtained in the current iteration ($r$) are compared against, and if necessary, appended to, a set of unique features $F_{unq}$ that consists of respective features constituting the $MD_{fwd}$ and $MD_{rev}$ obtained in earlier iterations (i.e., up to iteration $r$ - 1). After 'R' iterations, a plurality of forward models and a plurality of reverse models are generated for a predefined number of times using randomly partitioned internal training set and the internal test set. The iterations proceed while the value of R satisfies the following criteria -

(i)

$$R \leq R_{max}$$

(ii)

$$(|F_{unq}| \text{ after iteration R}) > (|F_{unq}| \text{ after iteration } R - R_{unq})$$

(iii)

$$|F_{unq}| \text{ after iteration no. } R \ <= \ Fet_{max}$$

Wherein, $R_{max}$ is a parameter indicating the maximum number of iterations allowed;
$R_{unq}$ is a parameter indicating the maximum number of iterations allowed without any cumulative increase in the number of unique features $|F_{unq}|$ in the models being generated in consecutive iterations; and
$Fet_{max}$ is a parameter indicating the maximum allowed value of $|F_{unq}|$ (i.e., the no. of unique features cumulated through the iterative process).

In an embodiment, the exemplary values of $R_{max}$, $R_{unq}$, and $Fet_{max}$ are 100, 10, 100 respectively for the present disclosure. Other values of these and other parameters here for finetuning and suitability for other datasets are within the scope of the present invention.

In the next step at 440, an ensemble of forward models is generated using the plurality of forward models and an ensemble of reverse models is generated using the plurality of reverse models. This is referred as an ensemble of forward models (ENS-$MD_{fwd}$)) and an ensemble of reverse models (ENS-$MD_{rev}$).

At step 442, the best models from each of these ensembles, i.e., the best of the forward models ($BMD_{fwd}$) and the best of the reverse models ($BMD_{rev}$) respectively, are identified using the model evaluation score *(MES)*.

If all models in an ensemble are weak models, the best model from the ensemble ($BMD$) is chosen by ranking the models based on their model evaluation scores and associated criteria. Also, if an ensemble contains more than one strong model, then only those strong models are considered for ranking based on their model evaluation scores and associated criteria as mentioned above, and the best model from the ensemble ($BMD$) is thereby chosen.

In the next step 444, a final single model ($FM_{single}$) is chosen as the ensemble classification model from amongst the best forward model and the best reverse model based on how they classify the individual samples from the training data. Once the best models from each of the ensemble of forward models and the ensemble of reverse models, i.e., the best of the forward models ($BMD_{fWd}$) and the best of the reverse models ($BMD_{rev}$) are identified, the final single model ($FM_{single}$) is chosen from amongst $BMD_{fwd}$ and $BMD_{rev}$ based on how well they can classify the individual training biological samples from the entire training set (*TR*). The AUC value for ROC curves for each of these two models are computed based on the predicted model scores for the training set (*TR*) samples and their pre-assigned classes (the first class (A) and the second class (B)). The model having the best AUC for ROC value is selected as the final single model ($FM_{single}$). If both $BMD_{fwd}$ and $BMD_{rev}$ have the same AUC value, $BMD_{fwd}$ is chosen as $FM_{single}$.

In an alternate implementation $FM_{single}$ can be chosen based whether $BMD_{fwd}$ or $BMD_{rev}$ obtains a higher *MCC* value while classifying the *TR* training biological samples. Once the $FM_{single}$ model has been chosen, for classification of any samples from a test set (*TS*) or any sample data received during actual deployment, the *FM_{single}* model is used after:

(a) appropriately transforming the features corresponding to the training biological sample being classified using the $\widetilde{Q1}_J,\ \widetilde{Q3}_J,\ \widetilde{Q2_{A_J}}$ and $\widetilde{Q2_{B_J}}$ values corresponding to the *FM_{single}* model,

(b) limiting the model score between a maximum of $CMS_{K_{max}}$ and a minimum of $CMS_{K_{min}}$ values corresponding to the *FM_{single}* model, and

(c) classification based on the model score using its corresponding threshold $T_{max}$

According to an embodiment of the disclosure, the ensemble of forward models (ENS-*MD_{fwd}*) and the ensemble of reverse models (ENS-*MD_{rev}*) are also evaluated for their collective classification efficiencies using an ensemble model scoring. In the ensemble scoring method, each of the models (*MD*) constituting an ensemble (*ENS*) are used to generate a model score (*MS*) for each of the samples from the entire *TR* set. For any specific training biological sample, the values of the features corresponding to the training biological sample are appropriately transformed using the $\widetilde{Q1}_J,\ \widetilde{Q3}_J,\ \widetilde{Q2_{A_J}}$ and $\widetilde{Q2_{B_J}}$ values corresponding to the model MD. The model scores (*MS*) are then transformed into scaled model scores (*SMS*) having values between -1 and +1, using the following procedure:

$$SMS = (MS - T_{max})/(CMS_{K_{max}} - T_{max}), \ \ldots\ldots\ when\ MS >= T_{max},$$

and

$$SMS = (MS - T_{max})/(T_{max} - CMS_{K_{min}}), \ \ldots\ldots\ when\ MS < T_{max},$$

Wherein, $T_{max}$, $CMS_{K_{max}}$, and $CMS_{K_{min}}$ values corresponding to the respective model is used.

Let $SMS_{avg}$ be the average of all *SMS* obtained using all models in ENS for a particular sample. When using Forward model [*ENS-MD_{fwd}*],

$$SMS_{avg} = SMS_{avg} * (+1)$$

If $SMS_{avg} >= 0$, sample is classified as the second class (B)
If $SMS_{avg} < 0$, sample is classified as the first class (A)
When using Reverse model [ENS-*MD_{rev}*]:

$$SMS_{avg} = SMS_{avg} * (-1)$$

If $SMS_{avg} > 0$, sample is classified as the second class (B)
If $SMS_{avg} <= 0$, sample is classified as the first class (A)

If all models in one of the ensembles are weak models, then the other one having (one or more) strong models is selected as a final ensemble model (*FM_{ens}*), and subsequently used for classification of any of training biological samples from a test set (*TS*) or any sample data received during actual deployment of the method, using the scoring and classification process mentioned in above paragraph. If both ensembles have constituent strong models, then both the ensembles are evaluated for their efficiency by scoring them on all individual samples in *TR*. The AUC value for ROC curves for each of these two ensembles are computed based on the predicted $SMS_{avg}$ for all the training set (*TR*) samples and their pre-assigned classes. The ensemble of models having the best AUC for ROC value is selected as the final ensemble model (*FM_{ens}*). In case both ENS-*MD_{fwd}* and ENS-*MD_{rev}* exhibit equal AUC values then ENS-*MD_{fwd}* is chosen as the final ensemble model (*FM_{ens}*). In an alternate implementation, *FM_{ens}* can be chosen based whether ENS-*MD_{fwd}* and ENS-*MD_{rev}* obtains a higher average MCC value for their respective constituent models while classifying the *TR* samples.

Thus, either the *FM_{single}* model or *FM_{ens}* ensemble of models is the binary classification model and can be used for classification of any of training biological samples from a test set (*TS*) or any training biological sample data received during actual deployment.

At step 212 of the method 200, the subject is stratified or categorized as one of (i) the responder to the therapy or (ii) the non-responder to the therapy, based on the model score obtained at step 210 of the method 200, through the assessment module 116. Further a predefined threshold value is used as a reference with which the model score is compared in the stratification. In an embodiment, if the model score is greater than or equal to the predefined threshold value, then the subject is stratified as the responder to the therapy. Similarly, if the model score is lesser than the predefined threshold value, then the subject is stratified as the non-responder to the therapy.

Hence, based on the model score, any biological sample from any subject can be evaluated for category of response of one or more microbiome-based drug/ therapy. A worked-out example/ complete workflow is described in later part of the disclosure. The method of the present disclosure can be utilized for the accurate stratification of individuals into responders and non-responders and clinical trials and therapeutic strategies can be effectively made based upon the responsiveness of individuals or a group of individuals with similar responsiveness profiles.

At step 214 of the method 200, a personalized recommendation is made to the subject for or against the therapy, through the recommendation module 118, based on the stratification on the responsiveness of the subject to the therapy obtained at step 212 of the method 200.

At step 216 of the method 200, the responsiveness of the subject to one or more therapies is determined and guiding a best therapy among the one or more therapies based on the determined responsiveness.

In an embodiment, the personalized recommendation includes utilizing a set of rules for the set of microbes that constitute the binary classification model to identify one or more personalized probiotic and antibiotic candidates that may be employed to ameliorate disease symptoms in the subject. In an embodiment, the microbes (organisms) contributing to generation of model score at step 210 are mapped to a predefined set of antibiotic and probiotic candidates and appropriate personalized targets for treatment and recommendation are identified accordingly.

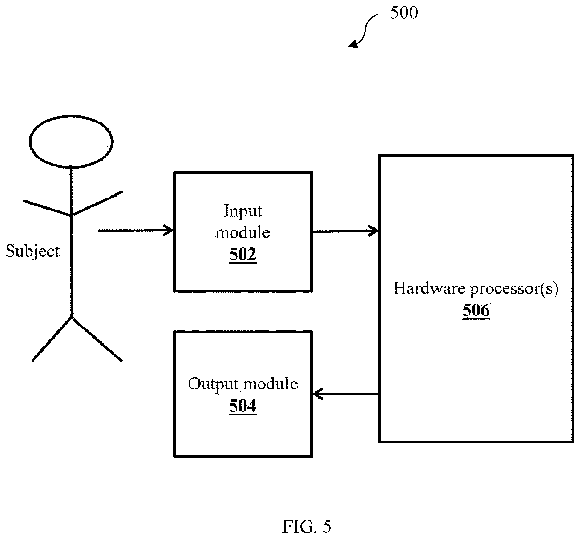

Further, a kit for stratification of the subject as one of (i) the responder to the therapy the (ii) the non-responder to the therapy. FIG. 5 illustrates an exemplary block diagram of a kit 500 for stratification of the subject as one of the responders or the non-responders to the therapy, according to some embodiments of the present disclosure. As shown in FIG. 5, the kit 500 includes an input module 502, one or more hardware processors 504 and an output module 506. The input module 502 is used for collecting a test biological sample from the subject for the stratification into one of (i) the responder to a therapy and (ii) the non-responder to the therapy. In an embodiment, the input module 502 may be a medium, a carrier, a set of mediums, or a set of carries that can hold the test biological sample.

The one or more hardware processors 506 are configured analyze the test biological sample using the steps of the method 200. In an embodiment, the one or more hardware processors 506 are equivalent or same that of the one or more hardware processors 106 of the system 100. The output module 506 is used for the stratification of the subject as one of (i) the responder to the therapy or (ii) the non-responder to the therapy, based on the analysis of the one or more hardware processors. In other words, the output module 506 is used for indicating on the presence or non-presence responsiveness of the subject to the provided therapy. In an embodiment, the output module 506 includes but are not limited to a display device, an indicator, a color indicator, or any other equipment that can show the result representation on the responsiveness of the subject.

According to embodiments of the present disclosure, the method proposed can be extended to identify an array of biomarkers/ signatures that determine the responsiveness for a panel of commonly administered pre/pro/synbiotics. Further, any new gut microbiome sample can be tested for the presence of these biomarkers/ signatures and then later be mapped to the microbiome-targeted intervention(s) specific for the identified biomarkers. This assist in the making and development of personalized/ precision medicine based upon the baseline microbiome of an individual. Such strategies when employed in clinical settings are configured to assist health-care providers to effectively screen/ treat/ manage individuals or a group of individuals with similar responsiveness profiles.

According to an embodiment of the disclosure, the method 200 can also be explained with the help of a working example as follows. Initially in step 1, a stool sample is obtained from an individual for whom we intend to ascertain the response to a prebiotic. In step 2, the raw abundances of various microbial taxonomic groups in the stool sample are quantified. Methodology for this involves extraction of microbial DNA contents from the collected stool sample followed by amplification and sequencing of either full-length or specific variable regions of the bacterial 16S rRNA marker genes using a next-generation sequencing platform or by using the multiplex qPCR-based quantification methodology. Table 2 below shows raw abundance of features in a test biological sample.

Table 2: Raw abundance of features in a test sample

| Features | Raw Abundance |
|---|---|
| g_Acinetobacter;s_guillouiae | 0 |

(continued)

| Features | Raw Abundance |
|---|---|
| g_Aquaspirillum;s_serpens | 0 |
| g_Bacteroides;s_plebeius | 330 |
| g_Bacteroides;s_uniformis | 153 |
| g_Haemophilus;s_parainfluenzae | 742 |
| g_Hyphomicrobium;s_sulfonivorans | 0 |
| g_Parabacteroides;s_distasonis | 63 |
| g_Plesiomonas;s_shigelloides | 0 |
| .. | .. |
| .. | .. |
| .. | .. |
| g_Rhodococcus;s_fascians | 0 |
| g_Rothia;s_mucilaginosa | 25 |

In step 3 the abundances values of various taxa in the sample are percent normalized. Table 3 shows percent normalized abundance of features in the test biological sample.

Table 3: Percent normalized abundance of features in the test sample

| Features | Normalized Abundance |
|---|---|
| g_Acinetobacter;s_guillouiae | 0 |
| g_Aquaspirillum;s_serpens | 0 |
| g_Bacteroides;s_plebeius | 0.13 |
| g_Bacteroides;s_uniformis | 0.06 |
| g_Haemophilus;s_parainfluenzae | 0.28 |
| g_Hyphomicrobium;s_sulfonivorans | 0 |
| g_Parabacteroides;s_distasonis | 0.02 |
| g_Plesiomonas;s_shigelloides | 0 |
| .. | .. |
| .. | .. |
| .. | .. |
| g_Rhodococcus;s_fascians | 0 |
| g_Rothia;s_mucilaginosa | 0.01 |

In step 4, from the normalized abundance table, abundances of only the subset of taxa which overlap with the list of two taxa that are provided against 'single best training model' are retained as mentioned below in Table 4.

Table 4: Model characteristics of features in the Single Best Training Model

| Features | g_Haemophilus;s_parainfluenzae | g_Rothia;s_mucilaginosa |
|---|---|---|
| Q1 | 0.04 | 0 |
| Q3 | 0.55 | 0.02 |
| $Q2_A$ | 0.22 | 0.01 |

(continued)

| Features | g_Haemophilus;s_parainfluenzae | g_Rothia;s_mucilaginosa |
|---|---|---|
| $Q2_B$ | 0.04 | 0.01 |
| Min Model Score | 0.33 | |
| Max Model Score | 0.95 | |
| Threshold | 0.65 | |
| Numerator/ Denominator Feature | Denominator | Denominator |
| Model Type | Forward | |

As an example, assume that the three taxa in the taxonomic abundance profile obtained by processing the stool sample (in the manner mentioned in Steps 1 and 2) had the following rarefied abundances:

Abundance of g_Haemophilus;s_parainfluenzae (i.e., feature 1 in training model) in collected stool sample: 0.28
Abundance of g_Rothia;s_mucilaginosa (i.e., feature 2 in training model) in collected stool sample: 0.01

In step 5, using $Q1$ and $Q3$ values corresponding to each training model feature in the single best model (as mentioned in Table 3), the transformation is applied, to above rarefied abundances, results in the following:

Transformed abundance ($F_{g\_Haemophilus;s\_parainfluenzae}$): 0.477679
Transformed abundance ($F_{g\_Rothia;s\_mucilaginosa}$): 0.384615

The transformed abundance of individual features as obtained above are then used appropriately in the candidate model equation ($CM_K$) (as replicated below), and numerator and denominator sums are computed. In this case, the values obtained are as follows:

Since Numerator sum = 0 and Denominator sum = 0.425110 in this case, a value of 1 is added to both numerator and denominator

$$\mathrm{CM_K} = \frac{\sum F_{numerator}}{\sum F_{denominator}} \dots \text{ when } F_{numerator} > 0 \text{ and } F_{denominator} > 0$$

or,

$$\mathrm{CM_K} = \frac{\sum F_{numerator}+1}{\sum F_{denominator}+1} \dots \text{ when either } F_{numerator} \text{ or } F_{denominator} = 0$$

Numerator sum: 1.000000
Denominator sum: 1.862294

In the step 6, the sample model score *(MS)* is computed next using above Numerator sum and Denominator sum. The sample model score *(MS)* is then transformed into scaled model score (*SMS*) (having values between -1 and +1, using following rules:

$$SMS = (MS - T_{max})/(CMS_{K_{max}} - T_{max}), \dots \text{ when } MS >= T_{max},$$

and

$$SMS = (MS - T_{max})/(T_{max} - CMS_{K_{min}}), \dots \text{ when } MS < T_{max},$$

Wherein, $T_{max}$, $CMS_{K_{max}}$, and $CMS_{K_{min}}$ values corresponding to the respective model is used.

For this purpose, the values of threshold: 0.654292,
Maximum model score: 0.946035,
Minimum model score: 0.333333 for single best model (as mentioned in Table 3) are employed.
Model score (*MS*): 0.536972
Scaled model score (*SMS*): -0.365529

In step 7, the *SMS* is then used for predicting the prebiotic response category of the individual from whom the stool sample was obtained. Since both forward model and reverse model are evaluated, wherein the final selected model is then used for classification or prediction. Here in this case, final selected single best model is a forward model, hence the final prediction score value is calculated as (*SMS* * +1).
Final pred_score is -0.365529, Since the value is <0, the prediction class is "A" i.e., responder category.

Following the same series of steps, if the value of *SMS* is greater than 0 then prediction class will be "B" and thus the prebiotic response category for the individual from whom the stool sample was obtained will be non-responder.

In step 8, similarly, for ensemble model, all the steps are repeated for all the single models in the ensemble and finally the average of all the Final prediction score is calculated using sample model scores (*SMS*) and the class prediction is done based on final average prediction score obtained for that sample.

It may be noted that the name and lineage of microbial groups changes from time to time due to changes in nomenclature, classification database etc. Therefore, any microbial 16S rRNA (or any other phylogenetic marker) gene sequences of >=97% sequence similarity and >=95% coverage with the corresponding 16S rRNA (or any other phylogenetic marker) gene sequences of the predetermined microbes (g_Haemophilus; s_parainfluenzae, and g_Rothia; s_mucilaginosa) will come also under the scope of this invention.

The sequence listing corresponding to the 16S rRNA gene of the set of microbial organisms belonging to the predetermined microbes are listed below. In one embodiment, at least one of the listed sequences, their fragments, and their upstream/downstream sequence (or sequence fragments) thereof may be utilized for determining the microbial abundance of each of the one or more predetermined microbes present in the test biological sample using the multiplex quantitative Polymerase Chain Reaction (qPCR) technique, from the microbial DNA, to obtain the microbial taxonomic profile associated with the test biological sample. In another embodiment, at least one of the listed sequences, their fragments, and their upstream/downstream sequence (or sequence fragments) thereof may be utilized for determining the microbial abundance of each of the plurality of microbes present in the test biological sample, using stretches of DNA sequences sequenced from the microbial DNA, to obtain the microbial taxonomic profile associated with the test biological sample.

>lcl|NZ_GL872342.1_rrna_56 [locus_tag=HMPREF9417_RS00035] [db_xref=RFAM: RF00177] [product=16S ribosomal RNA] [location=complement (52..1591)] [gbkey=rRNA] - Haemophilus parainfluenzae:

GAATTGAAGAGTTTGATCATGGCTCAGATTGAACGCTGGCGGCAGGCTTA
ACACATGCAAGTCGAACGGTAACATGAAGAAGCTTGCTTCTTTGATGACG
AGTGGCGGACGGGTGAGTAATGCTTGGGAATCTAGCTTATGGAGGGGGA
TAACTACGGGAAACTGTAGCTAATACCGCGTAGTATCGGAAGATGAAAG
TGTGGGACCTTCGGGCCACATGCCATAGGATGAGCCCAAGTGGGATTAGG
TAGTTGGTGAGGTAAAGGCTCACCAAGCCGACGATCTCTAGCTGGTCTGA
GAGGATGACCAGCCACACTGGGACTGAGACACGGCCCAGACTCCTACGG
GAGGCAGCAGTGGGGAATATTGCGCAATGGGGGCAACCCTGACGCAGCC
ATGCCGCGTGAATGAAGAAGGCCTTCGGGTTGTAAAGTTCTTTCGGTAGC
GAGGAAGGCATTTAGTTTAATAGACTAGGTGATTGACGTTAACTACAGAA
GAAGCACCGGCTAACTCCGTGCCAGCAGCCGCGGTAATACGGAGGGTGC
GAGCGTTAATCGGAATAACTGGGCGTAAAGGGCACGCAGGCGGTGACTT
AAGTGAGGTGTGAAAGCCCCGGGCTTAACCTGGGAATTGCATTTCATACT
GGGTCGCTAGAGTACTTTAGGGAGGGGTAGAATTCCACGTGTAGCGGTGA
AATGCGTAGAGATGTGGAGGAATACCGAAGGCGAAGGCAGCCCCTTGGG
AATGTACTGACGCTCATGTGCGAAAGCGTGGGGAGCAAACAGGATTAGA
TACCCTGGTAGTCCACGCTGTAAACGATGTCGATTTGGGGGTTGAGCTTT
AAGCTTGGCGCCCGTAGCTAACGTGATAAATCGACCGCCTGGGGAGTACG
GCCGCAAGGTTAAAACTCAAATGAATTGACGGGGGCCCGCACAAGCGGT
GGAGCATGTGGTTTAATTCGATGCAACGCGAAGAACCTTACCTACTCTTG
ACATCCAGAGAACATTCCAGAGATGGATTGGTGCCTTCGGGAACTCTGAG
ACAGGTGCTGCATGGCTGTCGTCAGCTCGTGTTGTGAAATGTTGGGTTAA
GTCCCGCAACGAGCGCAACCCTTATCCTTTGTTGCCAGCGATTCGGTCGG
GAACTCAAAGGAGACTGCCGGTGATAAACCGGAGGAAGGTGGGGATGAC
GTCAAGTCATCATGGCCCTTACGAGTAGGGCTACACACGTGCTACAATGG
CGTATACAGAGGGAAGCGAGAGTGCGAGCTGGAGCGAATCTCACAAAGT
ACGTCTAAGTCCGGATTGGAGTCTGCAACTCGACTCCATGAAGTCGGAAT

CGCTAGTAATCGCAAATCAGAATGTTGCGGTGAATACGTTCCCGGGCCTT
GTACACACCGCCCGTCACACCATGGGAGTGGGTTGTACCAGAAGTAGATA
GCTTAACCTTCGGGGGGGCGTTTACCACGGTATGATTCATGACTGGGGTG
AAGTCGTAACAAGGTAACCGTAGGGGAACCTGCGGTTGGATCACCTCCTT
A

>lcl|NC_013715.1_rrna_RMDY18_RS06240_44  [locus_tag=RMDY18_RS06240]  [db_xref=RFAM:  RF00177] [product=16S ribosomal RNA] [location=complement (1592165..1593691)] [gbkey=rRNA] - Rothia mucilaginosa:

TCAACGGAGAGTTTGATTCTGGCTCAGGACGAACGCTGGCGGCGTGCTTA

ACACATGCAAGTCGAACGATGAAGCCTAGCTTGCTAGGTGGATTAGTGGC

GAACGGGTGAGTAATACGTGAGTAACCTACCTTTAACTCTGGGATAAGCC

CGGGAAACTGGGTCTAATACCGGATACGACCAATCTCCGCATGGGGTGTT

GGTGGAAAGCGTTATGTAGTGGTTATAGATGGGCTCACGGCCTATCAGCT

TGTTGGTGAGGTAACGGCTCACCAAGGCGACGACGGGTAGCCGGCCTGA

GAGGGTGACCGGCCACACTGGGACTGAGACACGGCCCAGACTCCTACGG

GAGGCAGCAGTGGGGAATATTGCACAATGGGCGCAAGCCTGATGCAGCG

ACGCCGCGTGAGGGATGACGGCCTTCGGGTTGTAAACCTCTGTTAGCAGG

GAAGAAGAGAGATTGACGGTACCTGCAGAGAAAGCGCCGGCTAACTACG

TGCCAGCAGCCGCGGTAATACGTAGGGCGCGAGCGTTGTCCGGAATTATT

GGGCGTAAAGAGCTTGTAGGCGGTTTGTCGCGTCTGCTGTGAAAGGCCGG

GGCTTAACTCCGTGTATTGCAGTGGGTACGGGCAGACTAGAGTGCAGTAG

GGGAGACTGGAATTCCTGGTGTAGCGGTGGAATGCGCAGATATCAGGAG

GAACACCGATGGCGAAGGCAGGTCTCTGGGCTGTAACTGACGCTGAGAA

GCGAAAGCATGGGGAGCGAACAGGATTAGATACCCTGGTAGTCCATGCC

GTAAACGTTGGGCACTAGGTGTGGGGGACATTCCACGTTTTCCGCGCCGT

AGCTAACGCATTAAGTGCCCCGCCTGGGGAGTACGGCCGCAAGGCTAAA

ACTCAAAGAAATTGACGGGGGCCCGCACAAGCGGCGGAGCATGCGGATT

AATTCGATGCAACGCGAAGAACCTTACCAAGGCTTGACATATACTGGACC

GCATCAGAGATGGTGTTTCCCTTCGGGGCTGGTATACAGGTGGTGCATGG

TTGTCGTCAGCTCGTGTCGTGAGATGTTGGGTTAAGTCCCGCAACGAGCG

CAACCCTCGTTCTATGTTGCCAGCACGTTATGGTGGGGACTCATAGGAGA

CTGCCGGGGTCAACTCGGAGGAAGGTGGGGATGACGTCAAATCATCATG

CCCCTTATGTCTTGGGCTTCACGCATGCTACAATGGCCGGTACAGAGGGT

TGCGATACTGTGAGGTGGAGCTAATCCCTAAAAGCCGGTCTCAGTTCGGA

TTGGGGTCTGCAACTCGACCCCATGAAGTCGGAGTCGCTAGTAATCGCAG

ATCAGCAACGCTGCGGTGAATACGTTCCCGGGCCTTGTACACACCGCCCG

TCAAGTCACGAAAGTTGGTAACACCCAAAGCCGGTGGCCTAACCTTTTGG

AGGGAGCCGTCTAAGGTGGGATTGGCGATTGGGACTAAGTCGTAACAAG

GTAGCCGTACCGGAAGGTGCGGCTGGATCACCTCCTTT

The embodiments of present disclosure herein address unresolved problem related to stratification of any therapy specific to a person. The embodiment thus provides the method and the system for stratification of the subject as one of the responders and the non-responder to the therapy. The method proposed in the present disclosure analyzes the baseline microbiome of patients (i.e., before the administration of any drug/ therapy) and predetermines who is likely to respond to a treatment and who is not. The method proposed in the present disclosure utilizes a novel features/ biomarker/microbes for characterization of responders and non-responders (for a microbiome-based drug/ therapy) which are identified by a novel supervised machine learning algorithm. The method does not require any in-vitro or in-vivo experiments for the stratification of responders and non-responders of any drug/ therapy. The method does not require the measurements of disease markers for the stratification of responders and non-responders of any drug/ therapy.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.  A method (200) comprising:

    collecting a test biological sample from a subject for stratification into one of (i) a responder to a therapy and (ii) a non-responder to the therapy (202);
    extracting a microbial Deoxyribonucleic Acid (DNA), from the test biological sample, using a DNA extraction technique (204);
    performing, via one or more hardware processors, one of (i) determining a microbial abundance of each of one or more predetermined microbes present in the test biological sample using a multiplex quantitative Polymerase Chain Reaction (qPCR) technique, from the microbial DNA, and (ii) determining the microbial abundance of each of a plurality of microbes present in the test biological sample, using from stretches of DNA sequences sequenced from the microbial DNA, to obtain a microbial taxonomic profile associated with the test biological sample (206);
    normalizing, via the one or more hardware processors, the microbial taxonomic profile associated with the test biological sample, using a data normalization technique, to obtain the normalized microbial taxonomic profile associated with the test biological sample (208);
    determining, via the one or more hardware processors, a model score using a binary classification model, based on the normalized microbial taxonomic profile associated with the test biological sample (210); and
    stratifying, via the one or more hardware processors, the subject as one of (i) the responder to the therapy or (ii) the non-responder to the therapy, based on the model score (212).

2.  The method (200) as claimed in claim 1, further comprising making a personalized recommendation to the subject for or against the therapy, based on the stratification on the responsiveness of the subject to the therapy (214).

3.  The method (200) as claimed in claim 1, further comprising determining the responsiveness of the subject to one or more therapies and guiding a best therapy among the one or more therapies based on the determined responsiveness (216).

4.  The method (200) as claimed in claim 1, wherein the binary classification model is obtained by:

    collecting, a first set of training biological samples and a second set of training biological samples, from a plurality of subjects, at a first time-point and a second time-point respectively, wherein the first time-point indicates before an administration of the therapy and the second time-point indicates after the administration of the therapy (302);
    extracting, the microbial Deoxyribonucleic Acid (DNA) from each training biological sample present in the first set of training biological samples and the second set of training biological samples, using the DNA extraction technique (304);
    sequencing, the microbial DNA associated with each training biological sample present in the first set of training biological samples and the second set of training biological samples, using a sequencer, to obtain the stretches of DNA sequences associated with each training biological sample (306);
    determining, the microbial abundance of each of one or more microbes present in each training biological sample present in the first set of training biological samples and the second set of training biological samples, using the stretches of DNA sequences associated with each training biological sample, to obtain a microbial taxonomic profile associated with each training biological sample, wherein the microbial taxonomic profile comprises the microbial abundance of each of the one or more microbes corresponding to the set of microbial DNA sequences present in each training biological sample (308);

normalizing, the microbial taxonomic profile using the data normalization technique, to obtain the normalized microbial taxonomic profile associated with each training biological sample, wherein the normalized microbial taxonomic profile comprises the normalized microbial abundance of each of the one or more microbes (310);

obtaining, a metabolic functional profile associated with each training biological sample, using the corresponding normalized microbial taxonomic profile (312);

quantifying, differences in the metabolic functional profile associated with each subject, based on the metabolic functional profile associated with each of the first set of training biological samples and the corresponding second set of training biological samples, using a gut-health score (314);

assigning, a tag to each subject of the plurality of subjects as one of:

(i) the responder to the therapy if the subject is showing an improvement in a gut-health status at the second time-point as compared to the first time-point, and

(ii) the non-responder to the therapy if the subject is showing a deterioration or no change in the gut-health status at the second time-point as compared to the first time-point, wherein the gut-health status is evaluated based on the corresponding gut-health score (316);

obtaining, one or more features associated to each subject, from the corresponding microbial taxonomic profile associated with each training biological sample present in the first set of training biological samples, based on the tag assigned to each subject (318); and

training, a machine learning model, using the one or more features associated to each subject of the plurality of subjects, to obtain the binary classification model (320).

5. The method (200) as claimed in claim 3, wherein the gut-health score is determined based on the abundance of gut microbial pathways corresponding to metabolism of one or both of beneficial metabolites or harmful metabolites at the first time-point and the abundance of the gut microbial pathways corresponding to one or more of the beneficial metabolites and the harmful metabolites at the second time-point.

6. The method (200) as claimed in claim 3, wherein training the machine learning model, using the one or more features associated to each subject of the plurality of subjects, to obtain the binary classification model, comprises:

(i) tagging one of a first class or a second class to each of a plurality of training biological samples obtained from the first set of training biological samples based on the assignment of the tag to each subject of the plurality of subjects as one of (i) the responder to the therapy or (ii) the non-responder to the therapy (402);

(ii) generating a training data further comprising a plurality of microbial abundance profiles from the plurality of training biological samples from the first set, wherein each microbial abundance profile corresponds to each of the plurality of training biological samples and comprises of one or more features and respective abundance values, and wherein each feature in the associated microbial abundance profile corresponds to one of a plurality of microbial taxonomic groups present in the associated training biological sample (404);

(iii) partitioning the training data into an internal training set and an internal test set, based on a predefined first parameter (406);

(iv) randomly selecting a predefined number of subsets out of the internal training set based on a predefined second parameter, wherein each subset comprises of a randomly selected one or more features, and wherein each subset comprises a plurality of training biological samples having a proportionate part of the training biological samples belonging to the first class and the proportionate part of the training biological samples belonging to the second class (408);

(v) noting, for each selected subset, a distribution of the abundance values of each of the features across the plurality of training biological samples in the selected subset, and the distribution of the abundance values of each of the features across the training biological samples belonging to the first class in the selected subset and the training biological samples belonging to the second class in the selected subset (410);

(vi) calculating, from the noted distributions of each selected subset, a first quartile value $Q1$ and a third quartile value $Q3$ of the distribution of each of the features across each of the training biological samples in the selected subset (412);

(vii) calculating, for each selected subset, a second quartile value of the distribution of each of the features across the training biological samples belonging to the first class $Q2_A$ in the selected subset and the training biological samples belonging to the second class $Q2_B$ in the selected subset (414);

(viii) calculating $Q1$, $Q3$, $Q2_A$ and $Q2_B$ for each of a predefined number of subsets M(416);

(ix) calculating a median value for each of the $Q1$, $Q3$, $Q2_A$ and $Q2_B$ (418);

(x) performing a Mann-Whitney test to check whether the median value ($Q2_A$) of the feature in the training biological samples belonging to the first class is significantly different (p<0.1) as compared to the median value ($Q2_B$) of the associated feature in the training biological samples belonging to the second class (420);

(xi) shortlisting the features based on a first predefined criteria utilizing calculated median values and the Mann-Whitney test (422);

(xii) generating a set of features using the shortlisted features using a second predefined criteria, wherein the set of features are less than or equal to a predefined second criteria value (424);

(xiii) creating a plurality of combinations of the features present in the set of features to generate a plurality of candidate feature sets, wherein a number of the plurality of combinations of the features is equal to a minimum of two and a maximum of the predefined second criteria value (426);

(xiv) building a plurality of candidate models ($CM_K$) corresponding to each of the plurality of candidate feature sets (428);

(xv) calculating a model evaluation score *(MES)* corresponding to each of the plurality of candidate models (430);

(xvi) selecting a model having a highest *MES*, out of the plurality of candidate models as a best model, based on a first threshold ($T_{max}$), wherein the selected model is tagged as a forward model (432);

(xvii) swapping the tagging of the first class and the second class to each of the plurality of training biological samples present in the training data (434);

(xviii) identifying and subsequently tagging the model as a reverse model by repeating the steps (ii) through (xvi) for the training data obtained after the swapping (436);

(xix) generating a plurality of forward models and a plurality of reverse models by repeating step (ii) through (xviii) for a predefined number of times using randomly created partitions of internal training sets and corresponding internal test sets from the training data (438);

(xx) generating an ensemble of forward models (ENS-$MD_{fwd}$) using the plurality of forward models and an ensemble of reverse models (ENS-$MD_{rev}$) using the plurality of reverse models (440);

(xxi) identifying a best forward model and a best reverse model using the model evaluation score *(MES)* (442); and

(xxii) choosing a final single model ($FM_{single}$) from amongst the best forward models and the best reverse model, and a final ensemble classification model ($FM_{ens}$) from among the ensemble of forward models and the ensemble of reverse models, based on the classification of the individual training biological samples from the training data, as a binary classification model (444).

7. The method (200) as claimed in claim 5, further comprises:

classifying each of the set of shortlisted features using a second threshold value different from the first threshold ($T_{max}$); and

cumulating the results to construct a receiver operating characteristic curve (ROC) for each of the shortlisted features, wherein an area under the curve (AUC) of the ROC is indicative of utility of the feature to distinguish between the training biological samples belonging to the first class and the second class.

8. The method (200) as claimed in claim 5, wherein calculating the model evaluation score *(MES)* comprises:

transforming the values of the set of features as follows:

$$F_j' = 0 \dots\dots\dots\dots\dots \text{ if } F_j < \widetilde{Q1}_J$$

$$F_j' = 1 \dots\dots\dots\dots\dots \text{ if } F_j > \widetilde{Q3}_J$$

$$F_j' = 0.5 \dots\dots\dots\dots\dots \text{ if } \widetilde{Q1}_J = \widetilde{Q3}_J$$

$$F_j' = \frac{F_j - \widetilde{Q1}_J}{\widetilde{Q3}_J - \widetilde{Q1}_J} \dots\dots\dots\dots\dots \text{ if } \widetilde{Q1}_J < F_j < \widetilde{Q3}_J;$$

collating the features out of the set of features as a set of numerator features ($F_{numerator}$) if $\widetilde{Q2_{B_J}} > \widetilde{Q2_{A_J}}$, else, collating the features out of the set of features as a set of denominator features ($F_{denominator}$); constituting a ratio function for each of the candidate model as:

$$CM_K = \frac{\Sigma F_{numerator}}{\Sigma F_{denominator}} \ldots \text{ when } F_{numerator} > 0 \text{ and } F_{denominator} > 0$$

or,

$$CM_K = \frac{\Sigma F_{numerator}+1}{\Sigma F_{denominator}+1} \ldots \text{ when either } F_{numerator} \text{ or } F_{denominator} = 0$$

wherein, $\Sigma F_{numerator}$ represents the sum of values of all numerator features for a particular training biological sample, and,
wherein, $\Sigma F_{denominator}$ represents the sum of values of all denominator features for a particular training biological sample;

generating a candidate model score ($CMS_K$) for each of the training biological samples in the internal train set; removing the top 10 percentile and bottom 10 percentile scores as outliers from the set of scores $CMS_K$, and identifying maximum and minimum scores from the set $CMS_K$ as $CMS_{K_{max}}$ and $CMS_{K_{min}}$ respectively;
reclassifying the training biological samples in the internal train set by considering each score in the set $CMS_K$ as threshold, such that the training biological sample is classified into the second class if $CMS_K$ is more than or equal to the threshold, or the training biological sample is classified into the first class if $CMS_K$ is less than the threshold;
calculating Matthew's correlation coefficients *(MCC)* for each of the thresholds based on a comparison of the reclassified training biological sample and the original classes of the training biological samples, to evaluate how well each of the thresholds are able to distinguish between the training biological samples associated to the first class and the second class;
identifying the threshold as a first threshold ($T_{max}$) which provides maximum absolute *MCC* value;
discarding the candidate models for further evaluation if the maximum absolute *MCC* value is less than 0.4;
considering the ($|MCC_{max}|$) value as the 'train-MCC' value ($MCC_{train}$) for the model $CM_K$ and the first threshold ($T_{max}$) is used to classify the training biological samples in the internal-test set;
comparing the classification results on the training biological samples from the internal test set against the original classes of the training biological samples with pre-assigned labels, and the *MCC* for the model $CM_K$ and the threshold $T_{max}$ threshold on the internal train set is calculated ($MCC_{test}$); and
calculating a model evaluation score *(MES)* for candidate model $CM_K$ as: $MES = |(MCC_{train} + MCC_{test})| - |(MCC_{train} - MCC_{test})|$.

9. The method (200) as claimed in claim 5 further comprising evaluating collective classification efficiencies of the ensemble of forward models (ENS-MD$_{fwd}$) and the ensemble of reverse models (*ENS-MD$_{rev}$*), using an ensemble model scoring method, wherein a model scores (*MS*) corresponding to each of the ensemble is transformed into a scaled model scores (*SMS*) having values between -1 and +1, wherein,

$$SMS = (MS - T_{max})/(CMS_{K_{max}} - T_{max}), \ldots \text{ when } MS >= T_{max},$$

and

$$SMS = (MS - T_{max})/(T_{max} - CMS_{K_{min}}), \ldots \text{ when } MS < T_{max},$$

wherein, $T_{max}$, $CMS_{K_{max}}$ and $CMS_{K_{min}}$ values corresponding to the respective model.

10. The method (200) as claimed in claim 8 further comprising calculating an average of all $SMS$ ($SMS_{avg}$) obtained using all models in the ensemble, wherein

$SMS_{avg} = SMS_{avg} * (+1)$ while using the ensemble of forward models (ENS-$MD_{fwd}$),

If $SMS_{avg} >= 0$, training biological sample is classified as the second class; and
If $SMS_{avg} < 0$, training biological sample is classified as the first class; and
$SMS_{avg} = SMS_{avg} * (-1)$ while using the ensemble of reverse model (ENS - $MD_{rev}$),
If $SMS_{avg} > 0$, training biological sample is classified as the second class; and
If $SMS_{avg} <= 0$, training biological sample is classified as the first class.

11. The method (200) as claimed in claim 9 further comprising selecting a final ensemble model ($FM_{ens}$) using the calculated $SMS_{avg}$, wherein the binary classification model is one of: the final single model ($FM_{single}$) or an ensemble of more than one classification models ($FM_{ens}$).

12. The method (200) as claimed in claim 5, wherein the first predefined criteria is if a feature ($F_j$) is observed to have significantly ($p<0.1$) different median values in the first class compared to the second class in >70% of predefined

$$\widetilde{Q2_{A_J}} >= Q2_{min} \quad \text{or} \quad \widetilde{Q2_{B_J}} >= Q2_{min}$$

number of subsets, and if $\widetilde{Q2_{A_J}} >= Q2_{min}$ or $\widetilde{Q2_{B_J}} >= Q2_{min}$, $F_j$ is added to a set of shortlisted features ($SF$).

13. A kit for stratification of a subject as one of (i) a responder to a therapy the (ii) a non-responder to the therapy, comprising:

an input module for collecting a test biological sample from the subject for the stratification into one of (i) the responder to a therapy and (ii) the non-responder to the therapy;
one or more hardware processors configured to analyze the test biological sample using the method performed in any of the claim 1 to claim 12; and
an output module for displaying the stratification of the subject as one of (i) the responder to the therapy or (ii) the non-responder to the therapy, based on the analysis of the one or more hardware processors.

FIG. 1

200

Collect a test biological sample from a subject for stratification into one of (i) a responder to a therapy and (ii) a non-responder to the therapy **202**

Extract a microbial Deoxyribonucleic Acid (DNA), from the test biological sample, using a DNA extraction technique **204**

Perform one of (i) determining a microbial abundance of each of one or more predetermined microbes present in the test biological sample using a multiplex quantitative Polymerase Chain Reaction (qPCR) technique, from the microbial DNA, and (ii) determining the microbial abundance of each of a plurality of microbes present in the test biological sample, using from stretches of DNA sequences sequenced from the microbial DNA, to obtain a microbial taxonomic profile associated with the test biological sample **206**

Normalize the microbial taxonomic profile associated with the test biological sample, using a data normalization technique, to obtain the normalized microbial taxonomic profile associated with the test biological sample **208**

A

FIG. 2A

200

(A)

Determine a model score using a binary classification model, based on the normalized microbial taxonomic profile associated with the test biological sample **210**

Stratify the subject as one of (i) the responder to the therapy or (ii) the non-responder to the therapy, based on the model score **212**

Make a personalized recommendation to the subject for or against the therapy, based on the stratification on the responsiveness of the subject to the therapy **214**

Determine the responsiveness of the subject to one or more therapies and guiding a best therapy among the one or more therapies based on the determined responsiveness **216**

FIG. 2B

300

Collect a first set of training biological samples and a second set of training biological samples, from a plurality of subjects, at a first time-point and a second time-point respectively, wherein the first time-point indicates before an administration of the therapy and the second time-point indicates after the administration of the therapy **302**

Extract the microbial Deoxyribonucleic Acid (DNA) from each training biological sample present in the first set of training biological samples and the second set of training biological samples, using the DNA extraction technique **304**

Sequence the microbial DNA associated with each training biological sample present in the first set of training biological samples and the second set of training biological samples, using a sequencer, to obtain the stretches of DNA sequences associated with each training biological sample **306**

Determine the microbial abundance of each of one or more microbes present in each training biological sample present in the first set of training biological samples and the second set of training biological samples, using the stretches of DNA sequences associated with each training biological sample, to obtain a microbial taxonomic profile associated with each training biological sample, wherein the microbial taxonomic profile comprises the microbial abundance of each of the one or more microbes corresponding to the set of microbial DNA sequences present in each training biological sample **308**

Normalize the microbial taxonomic profile using the data normalization technique, to obtain the normalized microbial taxonomic profile associated with each training biological sample, wherein the normalized microbial taxonomic profile comprises the normalized microbial abundance of each of the one or more microbes **310**

(B)

FIG. 3A

(B)

Obtain a metabolic functional profile associated with each training biological sample, using the corresponding normalized microbial taxonomic profile **312**

Quantify differences in the metabolic functional profile associated with each subject, based on the metabolic functional profile associated with each of the first set of training biological samples and the corresponding second set of training biological samples, using a gut-health score **314**

Assign a tag to each subject of the plurality of subjects as one of: (i) the responder to the therapy if the subject is showing an improvement in a gut-health status at the second time-point as compared to the first time-point, and (ii) the non-responder to the therapy if the subject is showing a deterioration or no change in the gut-health status at the second time-point as compared to the first time-point, wherein the gut-health status is evaluated based on the corresponding gut-health score **316**

Obtain one or more features associated to each subject, from the corresponding microbial taxonomic profile associated with each training biological sample present in the first set of training biological samples, based on the tag assigned to each subject **318**

Train a machine learning model, using the one or more features associated to each subject of the plurality of subjects, to obtain the binary classification model **320**

FIG. 3B

400

Assign one of a first class tag or a second class to each of the samples in the collected plurality of training biological samples from first set **402**

Generate training data comprising of a plurality of microbial abundance profiles corresponding to each of the plurality of training biological samples from first set, wherein each microbial abundance profile corresponding to a training biological sample comprises of one or a plurality of features and respective abundance values of the features, and wherein each feature in the microbial abundance profile corresponds to one of a plurality of microbial taxonomic groups present in the training biological sample **404**

Partition the training data into an internal training set and an internal test set **406**

Randomly select a predefined number of subsets out of the internal training set, wherein each subset comprises of a randomly selected plurality of microbial abundance profiles corresponding to the training biological samples in the randomly selected subset, and wherein each subset comprises a proportionate part of samples belonging to the first class and the remaining samples belonging to the second class **408**

Note for each selected subset, a distribution of the abundance values of each of the features across the plurality of training biological samples in the selected subset, and the distribution of the abundance values of each of the features across the training biological samples belonging to the first class in the selected subset and the training biological samples belonging to the second class in the selected subset **410**

Calculate from the noted distributions of each selected subset, a first quartile value (Q1) and a third quartile value (Q3) of the distribution of each of the features across each of the plurality of training biological samples in the selected subset **412**

C

FIG. 4A

Calculate for each selected subset, a second quartile value of the distribution of each of the features across the training biological samples belonging to the first class (Q2_A) ) in the selected subset and the training biological samples belonging to the second class (Q2_B) in the selected subset **414**

Calculate Q1, Q3 Q2_A Q2_B for each of a predefined number of subsets (M) **416**

Calculate median value for all calculated Q1, median value for all calculated Q3, median value for all calculated Q2_A and median value for all calculated Q2_B **418**

Perform a Mann-Whitney test to test if a value of the feature is significantly different between the samples belonging to the first class and the samples belonging to the second class **420**

Shortlist the features based on a first predefined criteria utilizing calculated median values and the Mann-Whitney test **422**

Generate a set of features using the shortlisted features using a second predefined criteria, wherein the set of features are less than or equal to 15 **424**

Create a plurality of combinations of the features present in the set of features to generate corresponding plurality of candidate feature sets, wherein the plurality of combinations of features comprises a minimum of two and a maximum of 15 features **426**

D

FIG. 4B

$\left(D\right)$

Build a plurality of candidate models corresponding to each of the plurality of candidate feature sets **428**

Calculate a model evaluation score (MES) corresponding to each of the plurality of candidate models **430**

Select a model out of the plurality of candidate models as the best model using the highest model evaluation score wherein the selected model is tagged as a forward model **432**

Swap the tags assigned to the first class and the second class of the plurality of training biological samples present in the training data **434**

Identify a model as a reverse model by repeating the steps (**404** to **432**) for the training data obtained after swapping the tags **436**

Generate a plurality of forward models and a plurality of reverse models for a predefined number of times using randomly partitioned internal training set and the internal test set **438**

Generate an ensemble of forward models using the plurality of forward models and an ensemble of reverse models using the plurality of reverse models **440**

Identify a best forward model and a best reverse model using the model evaluation score **442**

Choose a final single model (FM_single) as the ensemble classification model from amongst the best forward model and the best reverse model based on how they classify the individual training biological samples from the training data **444**

FIG. 4C

500

Subject

Input
module
**502**

Output module
**504**

Hardware processor(s)
**506**

FIG. 5

Europäisches
Patentamt

European
Patent Office

Office européen
des brevets

## EUROPEAN SEARCH REPORT

**Application Number**

EP 24 16 4328

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 968 338 A1 (ATLAS BIOMED GROUP LTD [GB]) 16 March 2022 (2022-03-16) * the whole document * | 1-13 | INV. G16B20/00 G16B40/20 G16H50/20 |
| Y | SWADHA ANAND ET AL: "'GutFeel': an in silico method for predicting gut health status based on the metabolic functional capabilities of the resident microbiome", FEBS LETTERS, ELSEVIER, AMSTERDAM, NL, vol. 595, no. 13, 3 June 2021 (2021-06-03) , pages 1825-1843, XP071257203, ISSN: 0014-5793, DOI: 10.1002/1873-3468.14107 * the whole document * * in particular * * Results section * | 1-13 | |
| Y | LI PEISHUN ET AL: "Machine learning for data integration in human gut microbiome", MICROBIAL CELL FACTORIES, [Online] vol. 21, no. 1, 23 November 2022 (2022-11-23), XP093194159, ISSN: 1475-2859, DOI: 10.1186/s12934-022-01973-4 Retrieved from the Internet: URL:https://www.ncbi.nlm.nih.gov/pmc/artic les/PMC9685977/pdf/12934_2022_Article_1973 .pdf> [retrieved on 2024-08-09] * the whole document * * in particular * * page 5 - page 12 * | 1-13 | TECHNICAL FIELDS SEARCHED (IPC) G16B G16H |

The present search report has been drawn up for all claims

| Place of search | Date of completion of the search | Examiner |
|---|---|---|
| Munich | 13 August 2024 | Rákossy, Z |

EPO FORM 1503 03.82 (P04C01)

## ANNEX TO THE EUROPEAN SEARCH REPORT
## ON EUROPEAN PATENT APPLICATION NO.

EP 24 16 4328

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2024

| Patent document cited in search report | Publication date | Patent family member(s) | Publication date |
|---|---|---|---|
| EP 3968338 A1 | 16-03-2022 | EP 3968338 A1 | 16-03-2022 |
| | | RU 2724498 C1 | 23-06-2020 |
| | | WO 2020226535 A1 | 12-11-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321028610